# EUROPÄISCHE PATENTSCHRIFT

(11) **EP 3 618 798 B1**
(45) Veröffentlichungstag und Bekanntmachung des Hinweises auf die Patenterteilung: **21.08.2024**
(21) Anmeldenummer: 17721673.6
(22) Anmeldetag: 05.05.2017
(51) Int. Cl.: A61K 8/33, A61K 8/34, A61K 8/35, A61K 8/37, A61K 8/49, A61Q 1/14, A61Q 5/02, A61Q 5/12, A61Q 11/00, A61Q 13/00, A61Q 19/00, A61Q 19/10, C11D 3/16, C11D 9/22, C11D 17/00

(54) **STOFFMISCHUNG ZUR GERUCHSVERBESSERUNG**
COMPOSITION FOR ODOUR IMPROVEMENT
MÉLANGE DE SUBSTANCES POUR L'AMÉLIORATION DE L'ODEUR

(43) Veröffentlichungstag der Anmeldung: 11.03.2020
(60) Teilanmeldung: 24183017.3
(73) Patentinhaber: Symrise AG, 37603 Holzminden Niedersachsen (DE)
(72) Erfinder: SCHULZE, Nicole, 31084 Freden (DE); SCHMIDT, Claudia, 37619 Kirchbrak (DE)
(74) Vertreter: Fabry, Bernd
(86) Internationale Anmeldenummer: PCT/EP2017/060745
(87) Internationale Veröffentlichungsnummer: WO 2018/202311

(56) Entgegenhaltungen:
- EP-A2- 0 761 808
- WO-A1-2016/049389
- WO-A1-2017/046058
- DE-A1- 3 237 038
- US-A- 5 783 244
- US-A1- 2003 096 731
- US-A1- 2016 272 921
- YAMANISHI TEI ET AL: "Methyl Jasmonate and Lactones Including Jasmine Lactone in Ceylon Tea", AGRICULTURAL AND BIOLOGICAL CHEMISTRY, 1 May 1973 (1973-05-01), pages 1075 - 1078, XP55908851, Retrieved from the Internet <URL:https://www.jstage.jst.go.jp/article/bbb1961/37/5/37_5_1075/_pdf> [retrieved on 20220404], DOI: 10.1080/00021369.1973.10860801
- WALTER W ET AL: "Verbindungen des Kaffeearomas", vol. 9, no. 2-3, 1 March 1969 (1969-03-01), pages 123 - 147, XP009523968, ISSN: 0044-264X, Retrieved from the Internet <URL:http://link.springer.com/article/10.1007/BF02021495/fulltext.html> [retrieved on 19690301], DOI: 10.1007/BF02021495
- SHELLIE R ET AL: "Characterisation of lavender essential oils by using gas chromatography-mass spectrometry with correlation of linear retention indices and comparison with comprehensive two-dimensional gas chromatography", JOURNAL OF CHROMATOGRAPHY A, ELSEVIER, AMSTERDAM, NL, vol. 970, no. 1-2, 13 September 2002 (2002-09-13), pages 225 - 234, XP004386646, ISSN: 0021-9673, DOI: 10.1016/S0021-9673(02)00653-2

## Beschreibung

### GEBIET DER ERFINDUNG

Die Erfindung befindet sich auf dem Gebiet der oleochemischen Produkte und betrifft Stoffmischungen, die die sensorische Wahrnehmung der durch oleochemische Zubereitungen gebildeten und unerwünschten Fehlnoten verursachenden Stoffe reduziert oder verhindert, sowie ein Verfahren zu deren Herstellung sowie deren Verwendung.

### STAND DER TECHNIK

Oleochemische Produkte und Zubereitungen aus den Bereichen der Kosmetik, Haushalt, Mundpflegemitteln, Fine Fragrance enthalten Fette, Fettsäuren, Fettsäureester, Fettsäuresalze oder Fett- bzw. Fettsäure-basierte Inhaltsstoffe. Gemeinsam ist allen diesen Stoffen, dass sie einen typischen fettigen Geruch besitzen, der bei längerer Lagerung zudem weitere unangenehme Noten entwickelt. Ursache hierfür sind neben kurzkettigen freien Fettsäuren, die durch Verseifung der Triglyceride freigesetzt werden, insbesondere Aldehyde und andere Stoffe, die durch Oxidation entstehen.

Diese unerwünschten Fehlnoten wirken sich negativ auf den Geruch oder das Aroma des Produktes oder Zubereitung und dessen Qualität aus. Gängige verwendete Parfümierungen oder Düfte bzw. Aromen können diese Fehlnote nur bedingt verdecken und müssen ggf. in erhöhter Konzentration eingesetzt werden, was wiederum erhöhte Kosten zur Folge hat.

Eine Methode, dieses Problem zu lösen, besteht darin, geruchlich nicht einwandfreie Chargen einer Nachbehandlung, der so genannten Desodorierung, zu unterwerfen. Dabei werden die Rohstoffe mit heißem Wasserdampf behandelt, wobei die Geruchsträger zu einem großen Teil abdestilliert werden. Das Verfahren ist jedoch mit hohem Energieeintrag verbunden. Insbesondere dann, wenn es darum geht, möglichst preiswerte Fette und Öle zu verarbeiten, die naturgemäß den höchsten Anteil an den genannten unerwünschten Geruchsträgern aufweisen, ist diese Maßnahme ökonomisch gesehen natürlich konterproduktiv.

In manchen Fällen ist es auch möglich, den unangenehmen Geruch durch Zugabe eines Verkapselungsmittels einzuschließen, wie dies beispielsweise in WO 2009 131748 A1 beschrieben wird. Allerdings ist auch diese Maßnahme aus ökonomischer Sicht kaum zu realisieren. Bei dieser Lösung besteht die Möglichkeit, das Riechstoffe oder Aromastoffe der zugesetzten Aromatisierung oder Parfümierung durch das Verkapselungsmittel ebenfalls gebunden werden könnten und sich dadurch negativ auf den gewünschten Duft oder Geschmack auswirken kann.

Die gängigste Alternative besteht bislang darin, den olfaktorisch nicht einwandfreien Rohstoffen - oder den damit konfektionierten Endprodukten - Duftstoffe beizufügen, die den unangenehmen Geruch oder Aroma überdecken (maskieren) oder sich mit diesem in einer Art und Weise kombinieren, dass ein neuer Geruch oder Aroma entsteht, der weniger unangenehm ist. Auch dieses Verfahren ist aufwendig und teuer, da bislang stets erhebliche Mengen an solchen Maskierungsstoffen erforderlich gewesen sind.

Das Dokument EP 2 865 739 A1 offenbart die Verwendung von Mischungen mit Lactonen der Formel (la) oder (Ib) 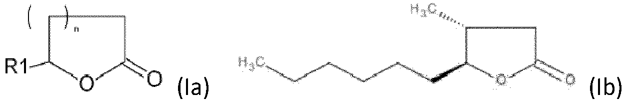 wobei R1 für einen linearen oder verzweigten, gesättigten oder ungesättigten, gegebenenfalls hydroxysubstituierten Kohlenwasserstoffrest mit 2 bis 22 Kohlenstoffatomen und 0 oder 1 bis 3 Doppelbindungen und n für die Zahlen 1 oder 2 steht, zur Maskierung von geruchlichen Fehlnoten in oleochemischen Zubereitungen.

Das Dokument WO 03/070871 A1 betrifft eine Zusammensetzung zum Entgegenwirken und/oder Maskieren von schlechtem Geruch, der sich während des Einweichens und/oder Handwaschen von Wäsche entwickelt. Die offenbarten Zusammensetzungen umfassen einen oder mehrere Schlechtgerüche unterdrückende Substanzen ausgewählt aus der Gruppe bestehend aus: Verbindungen der Formel (I), Aldehyd C-11 (Undecanal), Aldehyd C-11 Inter, Aldehyd C-12 Laurinsäure, Ethyl Rosate, Galbanum Öl, Hivertal, p-Methyl Kresol, Methylacetophenon, Patchouli-Öl, Undecavertol, Aldehyd C-11 Lenic, Allyl Cyclohexyl Propinate, Ambrettolide, Amylsalicylat, Damascon Delta, Dihydromyrcenol, Ethylvanillin, Florazon, Irisnitrile, Methyl lonone Gamma, Nonalacton Gamma, Pandanol, Rose Oxide L und Tridecen-2 nitril.

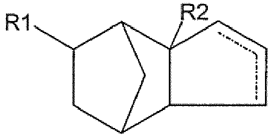

### Verbindungen der Formel (I) gemäß WO 03/070871 A1

Das Dokument US 2006/0207037 A1 offenbart ein Parfüm oder Riechstoffmischung, die optimiert ist auf die Überdeckung von stickstoffhaltigen Stoffe bedingte Schlechtgerüche, wie Ammoniak und substituierte Amine, die aber auch für andere Arten von Schlechtgerüchen wie Schweiß, Körpergeruch, Badzimmergerüche, Küchengerüche, etc angewendet werden kann. Weiterhin werden Duftstoffmischungen offenbart, die die Kombinationen von gamma-Undecalacton, gamma-Decalacton und Diethylphtalat (DEP) sowie die Kombination von gamma-Undecalacton, Geraniol, Linalool und Diethylphtalat (DEP) zeigen. Hauptverwendung dieser Riechstoffmischungen sind Haarfärbemittel, wobei sie auch für Produkte vom Haushaltsreiniger bis hin zu Kosmetika, umfassend Antiperspirationen, Deodorants, Selbstbräuner, Shampoos, Conditioner, Handlotion, Bodylotion und Dauerwelle verwendet werden können.

Im Dokument WO 2004/009750 A1 sind Duftstoffkomponenten, Gemische davon und Parfum-Zusammensetzungen zur Reduzierung oder Verhinderung von Körpergeruch beschrieben. Die identifizierten Duftstoffkomponenten verhindern die Herstellung riechender Steroiden, die von auf der Hautoberfläche vorhandenen Mikroorganismen hergestellt werden. Die Zusammensetzungen werden als Antimalodor-Wirkstoff als auch als Deo-Wirkstoff für Kosmetik bzw. Parfüm, Antiperspirantien und Deodorants beschrieben. Es wird die Kombination von gamma-Octalacton, gamma-Nonalacton, Geraniol, Dihydrolinalool und Tetrahydrogeraniol offenbart.

Das Dokument WO 96/04940 betrifft eine wässrige Zusammensetzung zur Herabsetzung der Wahrnehmung eines unangenehmen bzw. üblen Geruchs, die umfasst:
a) etwa 0,01 bis etwa 1% Parfüm, bezogen auf das Gewicht der Zusammensetzung;
b) gegebenenfalls, jedoch vorzugsweise, etwa 0,1 bis etwa 5% eines wasserlöslichen Cyclodextrins, bezogen auf das Gewicht der Zusammensetzung; c) gegebenenfalls, jedoch vorzugsweise, etwa 0,1 bis etwa 10% eines wasserlöslichen Metallsalzes, bezogen auf das Gewicht der Zusammensetzung; d) gegebenenfalls, jedoch vorzugsweise, etwa 0 bis etwa 3% eines Solubilisierungs-Hilfsmittels, bezogen auf das Gewicht der Zusammensetzung; und e) einen wässrigen Träger. Gamma-Decalacton und gamma-Dodecalacton befinden sich unter den verschiedenen Duftstoffen, die die Komponente a) enthalten kann. Die Zusammensetzung werden vorzugweise auf Textilien, insbesondere Kleider, aufgesprüht, ohne sie zu waschen oder trockenzureinigen.

Trotz der oben genannten Alternativen, die aus dem Stand der Technik bekannt sind, besteht weiterhin im Gebiet der oleochemischen Produkte ein starkes Bedürfnis nach neuen Stoffmischungen, die die oben genannten Nachteile überwinden, und die die Herstellung von qualitativ-hochwertigen Produkten und Zubereitungen ohne unerwünschten Geruchs- oder Aroma-Fehlnoten ermöglichen.

Weiterhin wirken sich diese unerwünschten Fehlnoten auch auf die Benutzung der Produkte oder Zubereitungen aus, denn beim Waschen, sprühen oder eincremen, versagen die gängigen Parfümierungen oder Aromatisierungen und die unerwünschten Fehlnoten treten besonders stark hervor.

Die Aufgabe der vorliegenden Erfindung hat somit darin bestanden, neue hochaktive Stoffmischungen zur Verfügung zu stellen, die schon in sehr geringen Konzentrationen in der Lage sind, die in oleochemischen Rohstoffen und in den damit hergestellten Endprodukten auftretenden unangenehmen Geruchs- oder Aroma-Fehlnoten, vorzugsweise Fehlnoten, die als stechend, kratzig, oxidiert, beißend, ranzig, fettig, animalisch, Ziege, vergoren, muffig, krautig und Erbrochenes von den Verbrauchern wahrgenommen werden, entgegenzuwirken, zu reduzieren oder zu maskieren.

Eine zweite Aufgabe der vorliegenden Erfindung hat somit darin bestanden, neue hochaktive Stoffmischungen zur Verfügung zu stellen, die in der Lage sind, den oben genannten unerwünschten Fehlnoten entgegenzuwirken, zu reduzieren oder zu maskieren, sowohl während der Benutzung als auch nach dem Abspülen der oleochemischen Zubereitungen bzw. Endprodukten, vor allem von der Hautoberfläche, die diese hochaktiven Stoffmischungen umfassen.

### BESCHREIBUNG DER ERFINDUNG

Beschrieben hierin sind Zubereitungen enthaltend
(i) eine Stoffmischung, enthaltend
   (a) ein, zwei oder mehrere Verbindungen ausgewählt aus der Gruppe bestehend aus
      (a1) Alkohol-Monoterpene der Formel (I) 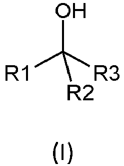 wobei
         R1 für H oder Methyl, R2 für H oder C₂-Alkenyl, und R3 für einen linearen oder verzweigten, gesättigten oder ungesättigten Kohlenwasserstoffrest mit 4 bis 10 Kohlenstoffatomen steht, sowie deren Enantiomere, Diastereomere, Razemate, Solvate und physiologisch verträglichen Salze,
         und/oder
      (a2) Bicyclische Epoxy-Monoterpene,
   (b) mindestens zwei Lactone der Formel (II) 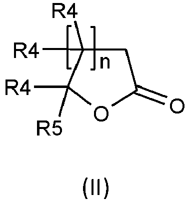 wobei
      R4 für H oder Methyl, R5 für einen linearen oder verzweigten, gesättigten oder ungesättigten Kohlenwasserstoffrest mit 2 bis 10 Kohlenstoffatomen und n für die Zahlen 1 oder 2 steht, sowie deren Enantiomere, Diastereomere und Razemate, und
   (c) ein, zwei oder mehrere Lösungsmittel ausgewählt aus der Gruppe bestehend aus Ethanol, Wasser, Dipropylenglykol (DPG), Diethylphtalat (DEP), Propylenglykol (PG), Isopropylmyristat (IPM), Isopropylpalmitat (IPP), Triethylcitrat (TEC), Triacetin (TRI), 1,2-Propandiol, 1,3-Propandiol, Propantiol, Pentandiol, Hexandiol, Octandiol, Decandiol (SymClariol^{®}), Dodecanol, 4-Hydroxy-Acetophenon (SymSave^{®} H), Glycerin, Butylenglykol, Pentylenglykol, Hexylenglykol, Decylenglykol, Propylencarbonat, Butylencarbonat, Glycerincarbonat, 2-Benzylheptanol, Laurylalkohol, Trimethyl-Hydroxypentyl-Isobutyrat, Glycerylcaprylat, Ethylhexylglycerin, Benzylbenzoat (BB),
      und gegebenenfalls
   (d) weitere Aroma- bzw. Duftstoffe ausgewählt aus der Gruppe bestehend aus 3-Phenylbutanal (Trifernal), Acetylmethylcarbinol, Anethol, Anisylacetat, Dihydroeugenol, Linalylformiat, 2-Methyldecanal, 2-Benzyl-2-methylbut-3-ene nitrile (Citrowanil^{®}B), 3-Hexenylacetat, Styralylacetat, Belanis, Citronellal, Cinnamylacetat, Rhubafuran, beta-Ionon, Anther, Prenylacetat, 2-Phenylpropanal, 4-(4-Hydroxyphenyl)butan-2-on (Frambinon^{®}), Ethylphenoxyacetat, Isoralderin, gamma-Terpinen, Limonen, Neocyclocitral, Methyllavenderketon, Styralylpropionat, Phenylethylpropionat, Limonenal, 4-Isopentylcyclohexanol (Symrose^{®}), 4-methyl-2-phenyl-3,6-dihydro-2H-pyran/4-methylen-2-phenyl-tetrahydropyran (Rosyrane super), Hydrocitronitril, Phenoxanol, Isoamylphenylacetat, Damascon, Silvial, Nectaryl, Ambroxid, Acetylpyrazin, Trimethylpyrazin, Isoamylacetat, Cresylmethylether para, Filbertone, Cyclohexylacetat, Heliotropin, Acetophenon, Anisaldehyd, Methylacetophenon para, Veratraldehyd, Methylanisat und Vertoprenal,
      und
(ii) Aldehyde der Formel (III) 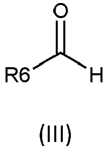 wobei R6 für einen gesättigten oder ungesättigten, linearen Kohlenwasserstoffrest steht,
   und/oder
(iii) freie Fettsäuren der Formel (IV). 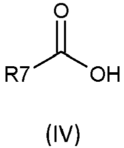 wobei R7 für einen linearen oder verzweigten, gesättigten Kohlenwasserstoffrest steht.

In einer bevorzugten Ausführungsform ist die hierin beschriebene Zubereitung eine oleochemische Zubereitung.

In einer bevorzugten Ausführungsform enthält die hierin beschriebene Zubereitung mindestens zwei, drei oder vier Stoffe der Gruppe (a).

In einerweiteren bevorzugten Ausführungsform ist die Komponente (a1) ausgewählt aus der Gruppe bestehend aus Linalool, Geraniol, Freesiol und Nerolidol sowie deren Enantiomere, Diastereomere, Razemate, Solvate und physiologisch verträglichen Salze.

In einer weiteren bevorzugten Ausführungsform sind die bicyclische Epoxy-Monoterpene (Komponente (a2)) Epoxy-p-Menthan-Derivate, insbesondere bevorzugt sind die Verbindungen 1,4-Cineol und 1,8-Cineol.

In einer bevorzugten Ausführungsform enthält die hierin beschriebene Zubereitung mindestens zwei Lactone - gegebenenfalls drei oder vier - ausgewählt aus der Gruppe bestehend aus der Lactone der Formel (II) sowie deren Enantiomere, Diastereomere und Razemate, wobei R4 für H, R5 für lineare und gesättigte Kohlenstoffreste mit 2 bis 10 Kohlenstoffatomen n für die Zahlen 1 oder 2 steht, besonders bevorzugt mit R4 = H und R5 = lineare und gesättigte Kohlenstoffreste mit 2 bis 8 Kohlenstoffatomen und n = 1 oder 2.

In einer weiteren bevorzugten Ausführungsform enthält die hierin beschriebene Zubereitung mindestens zwei Lactone - gegebenenfalls drei oder vier - ausgewählt aus der Gruppe bestehend aus gamma-Dodecalacton, gamma-Decalacton, gamma-Undecalacton, delta- Decalacton, delta- Dodecalacton, 4-Methyl-gamma-Nonalacton, 3-Methyl-gamma-Decalacton, gamma-Nonalacton, delta-Nonalacton, gamma-Octalacton, delta-Octalacton, gamma-Heptalacton, delta-Heptalacton, gamma-Hexalacton und delta-Hexalacton, bevorzugt gamma-Dodecalacton, gamma-Decalacton, delta-Decalacton und delta-Dodecalacton.

In einer bevorzugten Ausführungsform sind die Aldehyde der Formel (III) aus der Gruppe bestehend aus Hexanal, Heptanal, Octanal, Nonanal, Decanal, Hexenal, Heptenal, Octenal, Nonenal, Decenal, Undecenal, Dodecenal, Hexadienal, Heptadienal, Octadienal, Nonadienal, Decadienal, Undecandienal, Dodecandienal, Octatrienal, Nonatrienal, Decatrienal, Undecatrienal, Dodecatrienal, insbesondere bevorzugt Hexanal, Octanal, Nonanal, Hexenal, Heptenal, Octenal, Heptadienal Octadienal, Decadienal und Decatrienal ausgewählt.

In einer weiteren bevorzugten Ausführungsform sind die freie Fettsäuren der Formel (IV) aus der Gruppe bestehend aus Butansäure, Pentansäure, Isovaleriansäure, Hexansäure, Heptansäure, Octansäure, Nonansäure, Decansäure, Undecansäure und Dodecansäure, insbesondere bevorzugt Hexansäure, Octansäure und Decansäure ausgewählt.

In einer anderen bevorzugten Ausführungsform sind die Aldehyde der Formel (III) aus der Gruppe bestehend aus Hexanal, Heptanal, Octanal, Nonanal, Decanal, Hexenal, Heptenal, Octenal, Nonenal, Decenal, Undecenal, Dodecenal, Hexadienal, Heptadienal, Octadienal, Nonadienal, Decadienal, Undecandienal, Dodecandienal, Octatrienal, Nonatrienal, Decatrienal, Undecatrienal, Dodecatrienal, insbesondere bevorzugt Hexanal, Octanal, Nonanal, Hexenal, Heptenal, Octenal, Heptadienal, Octadienal, Decadienal und Decatrienal, und
die freie Fettsäuren der Formel (IV) aus der Gruppe bestehend aus Butansäure, Pentansäure, Isovaleriansäure, Hexansäure, Heptansäure, Octansäure, Nonansäure, Decansäure, Undecansäure und Dodecansäure, insbesondere bevorzugt Hexansäure, Octansäure und Decansäure ausgewählt.

In einer bevorzugten Ausführungsform ist die Komponente (c) der hierin beschriebenen Zubereitung ein, zwei oder mehrere Lösungsmittel ausgewählt aus der Gruppe bestehend aus Ethanol, Wasser, Dipropylenglykol (DPG), Diethylphtalat (DEP), Propylenglykol (PG), Isopropylmyristat (IPM), Isopropylpalmitat (IPP), Triethylcitrat (TEC), Triacetin (TRI), 1,2-Propandiol, 1,3-Propandiol, Propantiol, Pentandiol, Hexandiol, Octandiol, Decandiol (SymClariol^{®}), Dodecanol, 4-Hydroxy-Acetophenon (SymSave^{®} H) und Benzylbenzoat (BB).

In einer weiteren bevorzugten Ausführungsform enthält die hierin beschriebene Zubereitung einen oder mehrere Aroma- bzw. Duftstoffe der Gruppe (d), wobei diese aus der Gruppe aus 3-Phenylbutanal (Trifernal), Acetylmethylcarbinol, 2-Methyldecanal, 2-Benzyl-2-methylbut-3-ene nitrile (Citrowanil^{®}B), 3-Hexenylacetat, Styralylacetat, Rhubafuran, Anther, Prenylacetat, Styralylpropionat, Isoamylphenylacetat, beta-Damascon, Isoamylacetat, Citronellol und Cyclohexylacetat ausgewählt ist/sind.

Die hierin beschriebene Zubereitung kann mit zusätzlichen Duft- oder Aromastoffe kombiniert werden.

Hierin beschrieben ist daher auch eine Duft- oder Aromazusammensetzung, enthaltend die oben beschriebene Zubereitung in Kombination mit zusätzlichen Duft- oder Aromastoffe der Gruppe (e), die in oleochemischen Zubereitungen oder Endprodukten eingearbeitet werden kann.

Duft- bzw. Aromastoffe der Gruppe (e) sind Stoffe, die durch z.B. europäische oder amerikanische Rechtsprechung, z.B. die Verordnung (EG) Nr. 2232/96 des Europäischen Parlaments und des Rates und der Positivliste gemäß der Durchführungsverordnung (EU) Nr. 872/2012 der Kommission, definiert sind, und die Stoffe, die in *"Riechstoffe"* von Steffen Arctander, in "Perfume and Flavor Chemicals" vom Eigenverlag, Montclair, N. J. 1969 sowie in "Common Fragrance and Flavor Materials" von H. Surburg, J. Panten, 5th Edition, Wiley-VCH, Weinheim 2006 aufgeführt sind.

Beispiele für Duft-und Aromastoffe der Gruppe (e) sind
- Extrakte aus natürlichen Rohstoffen wie Etherische Öle, Concretes, Absolues, Resine, Resinoi-de, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniu-möl; Grapefruitöl; Guajakholzöl; Gurjunbalsam; Gurjunbalsamöl; Helichrysum-Absolue; He-lichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmel-öl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen
- Ester wie z.B. Ethylbutyrat, Allylcapronat, Benzylacetat, Methylsalicylat, Nerylacetat, Geranylacetat, Allylcapronat, Anisylformiat, Butylbutyrat, Butylcapronat, Butylidenphthalid, Benzylbenzoat, Citronellylacetat, Dimethylanthranilat, Dimethylanthranilat, Ethoxyethylacetat, Ethylbutyrat, Ethylcaprinat, Ethylcapronat, Ethylcrotonat, Ethyl-isobutyrat, Ethylisovalerianat, Ethyllactat, Ethylmethylbutyrat, Ethylpropionat, Ethylheptylat, Methyldihydrojasmonat (z.B. Hedion^{®}), cis-2-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, Isoamylisovalerianat, Isobutylbutyrat, Linalylacetat, Methylanthranilat, 2-Methylbutylacetat, Methylcapronat, Methylcinnamat, Methyljasmonat, 2-Methylmethylbutyrat, Methylthiobutyrat, 3-Methylthiohexylacetat, Nerylacetat, 1-Octylacetat, 3-Octylacetat, Phenylethylacetat, Phenylethylisovalerianat, Propylbutyrat, Ethylvanillinisobutyrat, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, Methylsalicylat, Methylsorbat;
- Alkohole wie z.B. Octenol, cis-3-Hexanol, Benzylalkohol, Phenylethylalkohol, Eugenol, cis-3-Hexenol, Isoamylalkohol, Methylbutanol, 3,1-Methylthiohexanol, 2,4-Nonadienol, 2,6-Nonadienol, 2,4-Nonadienol, 2-Octanol, 3-Octanol, 1,3-Octenol, Phenylethylalkohol, Phenylethylalkohol, Zimtalkohol;
- Aldehyde wie z.B. Acetaldehyd, Isobutyraldehyd, Nonadienal, 3-Phenylacetaldehyd, Benzaldehyd, Grapefruitaldehyd, Isobutyraldehyd, 5-Methylfurfural, Paraldehyd, Piperonal, Propionaldehyd, Vanillin, Ethylvanillin, Divanillin, Phenylacetaldehyd, Methional, Zimtaldehyd;
- Ketone wie z.B. Menthon, alpha-lonon, beta-lonon, 4-(p-Hydroxyphenyl)-2-butanon, 2-Heptanon, 3-Heptanon, 4-Heptanon, para-Hydroxybenzylaceton, 3,2,2-Methylcyclopentenolon, 6,5,2-Methylheptenon, Nootkaton, Pentandion, 4-(p-Hydroxyphenyl)-2-butanon;
- Ether wie z.B. 4-Hydroxy-5-methylfuranon, 3-Hydroxy- 4,5-dimethyl-2-(5H)-furanon, 2,5-Dimethyl-3-hydroxy-2(3H)-furanon, 2(5)-Ethyl-4- hydroxy-5(2)-methyl-3(2H)-furanon, p-Methoxybenzaldehyd, Guajacol, Methoxyvinylphenol, Ethylfuraneol, Ethylguajakol, Isoeugenolmethylether, Menthofuran, 2,5-Dimethyl-4-hydroxy-3(2H)-furanon, Homo-furaneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon, 1,1-Dimethoxy-2,2,5-trimethyl-4-hexan;
- Acetale wie z.B. Acetaldehyddiethylacetal; Heliotropindiethylacetal;
- Lactone wie z.B. Ethylenbrassylat, Globalide^{®}, Ambrettolid, Massoilacton, Tuberolacton, 4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;
- Terpene wie z.B. Citronellol; Nerol; Lavadulol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
- Terpenaldehyde wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton sowie die Dimethyl- und Diethylacetale von Geranial, Neral, 7-Hydroxy-3,7-dimethyloctanal; Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
- cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
- Sulfide und Disulfide wie z.B. Dimethylsulfid, Difurfuryldisulfid, Methylthiopropanal;
- Thiole wie z.B. Methylfuranthiol, Benzothiazol, Isopropylmethylthiazol, Sulfurol, 8,3-Thiomenthanon, 4,4,2-Thiomethylpentanon, 2,4,5-Trimethylthiazol, 2-Acetylthiazol, 2,4-Dimethyl-5-ethylthiazol;
- Pyrazine und Pyrroline wie z.B. Methylpyrazin, Acetylpyrazin, 2-Propionylpyrrolin, 2-Acetylpyrrolin, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Pentylpyridin
- cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
- cyclischen und cycloaliphatischen Ether wie z.B. Cedrylmethylether; Cyclododecyl-methylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 7-Cyclohexadecen-1-on; 8-Cyclohexadecen-1-on; 9-Cycloheptadecen-1-on; Cyclo-pentadecanon; Cyclohexadecanon;
- cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- cycloaliphatischen Ketone wie z.B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
- Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
- Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
- araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethyl-acetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
- aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
- aromatischen und araliphatischen Ketone wie z.B. 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
- aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphe-nylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-lsopropylchinolin; 6-lsobutylchinolin; 6-sec.-Butylchinolin; Indol; Skatol; 2-Methoxy-3-isopropylpyrazin; 2-Isobutyl-3-methoxypyrazin;
- Phenole, Phenylether und Phenylester wie z.B. Estragol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
- heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
sowie (hier nicht explizit genannte) Stereoisomere, Enantiomere, Stellungsisomere, Diastereomere, cis/trans-Isomere bzw. Epimere dieser Substanzen.

In einer bevorzugten Ausführungsform ist die Duft- oder die Aromazusammensetzung eine flüssige oder feste Aromazusammensetzung.

Es ist ebenso ein weiterer Gegenstand der vorliegenden Erfindung die Verwendung der hierin beschriebenen Zubereitung als Fine Fragrance oder Parfüm.

Ein Gegenstand der vorliegenden Erfindung ist die Verwendung eine Stoffmischung, enthaltend
(a) ein, zwei oder mehrere Monoterperne ausgewählt aus der Gruppe, die gebildet wird von Linalool, Geraniol, Freesiol und Nerolidol,
(b) mindestens zwei Lactone ausgewählt aus der Gruppe, die gebildet wird von gamma-Dodecalacton, gamma-Decalacton, delta-Decalacton und delta-Dodecalacton;
   (c1) Ethanol und/oder
   (c2) ein, zwei oder mehrere Lösungsmittel ausgewählt aus der Gruppe, die gebildet wird von Dipropylenglykol, Diethylphtalat, Propylenglykol, Isopropylmyristat, Isopropylpalmitat, Triethylcitrat, Triacetin, 1,2-Propandiol, 1,3-Propandiol, Propantriol, Pentandiol, Hexandiol, Octandiol, Decandiol, Dodecanol, 4-Hydroxyacetophenon, und Benzylbenzoatzur Reduzierung und/oder Maskierung von unerwünschten Fehlnoten in oleochemischen Zubereitungen, wobei die unerwünschten Fehlnoten in der oleochemischen Zubereitungen durch
      (i) Aldehyde der Formel (III) 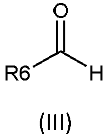 wobei R6 für einen gesättigten oder ungesättigten, linearen Kohlenwasserstoffrest mit 3 bis 10 Kohlenstoffatomen steht, und/oder
      (ii) freie Fettsäuren der Formel (IV) 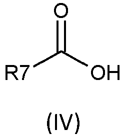 wobei R7 für einen linearen oder verzweigten, gesättigten Kohlenwasserstoffrest mit 3 bis 12 Kohlenstoffatomen steht,
         hervorgerufen werden,
         und gegebenenfalls
(d) weitere Aroma- bzw. Duftstoffe ausgewählt aus der Gruppe bestehend aus 3-Phenylbutanal (Trifernal), Acetylmethylcarbinol, Anethol, Anisylacetat, Dihydroeugenol, Linalylformiat, 2-Methyldecanal, 2-Benzyl-2-methylbut-3-ene nitrile (Citrowanil^{®}B), 3-Hexenylacetat, Styralylacetat, Belanis, Citronellal, Cinnamylacetat, Rhubafuran, beta-lonon, Anther, Prenylacetat, 2-Phenylpropanal, 4-(4-Hydroxyphenyl)butan-2-on (Frambinon^{®}), Ethylphenoxyacetat, Isoralderin, gamma-Terpinen, Limonen, Neocyclocitral, Methyllavenderketon, Styralylpropionat, Phenylethylpropionat, Limonenal, 4-Isopentylcyclohexanol (Symrose^{®}), 4-methyl-2-phenyl-3,6-dihydro-2H-pyran/4-methylen-2-phenyl-tetrahydropyran (Rosyrane super), Hydrocitronitril, Phenoxanol, Isoamylphenylacetat, Damascon, Silvial, Nectaryl, Ambroxid, Acetylpyrazin, Trimethylpyrazin, Isoamylacetat, Cresylmethylether para, Filbertone, Cyclohexylacetat, Heliotropin, Acetophenon, Anisaldehyd, Methylacetophenon para, Veratraldehyd, Methylanisat und Vertoprenal.

Überraschenderweise wurde gefunden, dass die erfindungsgemäße Stoffmischung in sehr geringen Konzentrationen in der Lage ist, die wahrgenommene Intensität von unangenehmen und unerwünschten Fehlnoten in einer Vielzahl von oleochemischen Formulierungen zu verringern bzw. zu maskieren, insbesondere Geruchs- oder Aroma-Fehlnoten, die als stechend, kratzig, oxidiert, beißend, ranzig, fettig, animalisch, Ziege, vergoren, muffig, krautig und Erbrochenes von den Testpersonen wahrgenommen werden, wie die experimentellen Daten der Tabelle 2 des Beispiels 1 belegen.

Darüber hinaus wurde überraschenderweise gefunden, dass die erfindungsgemäße Stoffmischung ihre Wirkung auch während der Phasen der Benutzung weiter entfaltet, sogar nach Abspülung der erfindungsgemäßen Stoffmischung bzw. der Zubereitung enthaltend die erfindungsgemäße Stoffmischung, wie die experimentelle Daten der Tabellen 3 und 4 des Beispiels 1 belegen. So wurde überraschenderweise gefunden, dass Zubereitungen enthaltend die erfindungsgemäße Stoffmischung die Überdeckung der unerwünschten Fehlnoten auch während der Anwendung, also beim Hände bzw. Haare waschen und nach dem Abspülen, bewirken, im Gegensatz zu normalen Parfümierungen bzw. Aromatisierungen bei dieser Dosierung.

Es hat sich herausgestellt, dass die erfindungsgemäße Stoffmischung besonders geeignet ist, um unerwünschten Fehlnoten zu maskieren, überdecken oder zu vermindert, die durch Aldehyde der Formel (III) 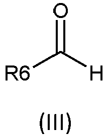
wobei R6 für einen gesättigten oder ungesättigten, linearen Kohlenwasserstoffrest steht, bevorzugt mit einer Kettenlänge von 3 bis 10 Kohlenstoffatomen,
und/oder freie Fettsäuren der Formel (IV) 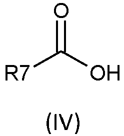
wobei R7 für einen linearen oder verzweigten, gesättigten Kohlenwasserstoffrest steht, bevorzugt eine Kettenlänge von 3 bis 12 Kohlenstoffatomen
hervorgerufen werden.

So wurde überraschenderweise gefunden, dass die erfindungsgemäße Stoffmischung bzw. die Kombination der Komponente (a), (b) und (c) und gegebenenfalls die Komponente (d) eine synergistische Wirkung aufweist. Es hat sich also herausgestellt, dass die erfindungsgemäße Kombination deutlich besser die unerwünschten Fehlnoten überdeckt als die Stoffe der Gruppe (a), (b) und (c), und optional der Gruppe (d) allein (synergistische Wirkung) und die von den Aldehyden der Formel (III) bzw. von Aldehyden der Formel (III) und Fettsäuren der Formel (IV) verursachten Fehlnoten in Kombination besser überdecken als die Gruppen für sich allein, wie die experimentelle Daten der Tabelle 2 des Beispiels 1 belegen.

Die von Aldehyden der Formel (III) und Fettsäuren der Formel (IV) verursachten Fehlnoten werden als stechend, kratzig, oxidiert, beißend, ranzig, fettig, animalisch, Ziege, vergoren, muffig, krautig und Erbrochenes von den Testpersonen beschrieben bzw. wahrgenommen.

Es hat sich herausgestellt, dass die erfindungsgemäße Stoffmischung besonders geeignet ist, um unerwünschten Fehlnoten zu maskieren, überdecken oder zu vermindert, die durch Aldehyde der Formel (III) und/oder freie Fettsäuren der Formel (IV),
wobei die Aldehyde der Formel (III) aus der Gruppe bestehend aus Hexanal, Heptanal, Octanal, Nonanal, Decanal, Hexenal, Heptenal, Octenal, Nonenal, Decenal, Undecenal, Dodecenal, Hexadienal, Heptadienal, Octadienal, Nonadienal, Decadienal, Undecandienal, Dodecandienal, Octatrienal, Nonatrienal, Decatrienal, Undecatrienal, Dodecatrienal insbesondere bevorzugt Hexanal, Octanal, Nonanal, Hexenal, Heptenal, Octenal, Heptadienal Octadienal, Decadienal und Decatrienal ausgewählt sind,
   und
die freie Fettsäuren der Formel (IV) aus der Gruppe bestehend aus Butansäure, Pentansäure, Isovaleriansäure, Hexansäure, Heptansäure, Octansäure, Nonansäure, Decansäure, Undecansäure und Dodecansäure, insbesondere bevorzugt Hexansäure, Octansäure und Decansäure ausgewählt sind.

Daher ist Gegenstand der vorliegenden Erfindung die Verwendung der erfindungsgemäße Stoffmischung zur Reduzierung und/oder Maskierung von unerwünschten Geruchs- oder Aroma-Fehlnoten in oleochemischen Zubereitungen, insbesondere von unerwünschten Fehlnoten, die durch Aldehyde der Formel (III) und/oder freie Fettsäuren der Formel (IV) wie oben definiert, hervorgerufen werden.

In einer bevorzugten Ausführungsform enthält die erfindungsgemäße Stoffmischung mindestens zwei, drei oder vier Stoffe der Gruppe (a).

Es hat sich herausgestellt, dass als Komponente (a1) die Verbindungen Linalool, Geraniol, Freesiol und Nerolidol sowie deren Enantiomere, Diastereomere, Razemate, Solvate und physiologisch verträglichen Salze besonders geeignet sind, um die unerwünschten Fehlnoten der Aldehyde der Formel (III) und/oder der freien Fettsäuren der Formel (IV) zu maskieren, insbesondere die von der oben genannten konkreten Aldehyde der Formel (III) und/oder der konkreten freien Fettsäuren der Formel (IV) unerwünschten Fehlnoten.

Bezüglich der Komponente (b) enthält die erfindungsgemäße Stoffmischung mindestens zwei, gegebenenfalls drei oder vier Lactone der Formel (II).

Die erfindungsgemäße Stoffmischung enthält mindestens zwei Lactone - gegebenenfalls drei oder vier - ausgewählt aus der Gruppe bestehend aus gamma-Dodecalacton, gamma-Decalacton, delta-Decalacton und delta-Dodecalacton.

Es hat sich herausgestellt, dass als Komponente (b) gamma-Decalacton, delta-Decalacton, gamma-Dodecalacton und delta- Dodecalacton besonders geeignet sind, um die unerwünschten Fehlnoten der Aldehyde der Formel (III) und/oder der freien Fettsäuren der Formel (IV) zu maskieren, insbesondere die von der oben genannten konkreten Aldehyde der Formel (III) und/oder der konkreten freien Fettsäuren der Formel (IV) unerwünschten Fehlnoten.

Bezüglich der Komponente (c) hat es sich herausgestellt, dass die Kombination von Ethanol mit einem, zwei oder mehreren Lösungsmitteln ausgewählt aus der Gruppe bestehend aus Dipropylenglykol (DPG), Diethylphtalat (DEP), Propylenglykol (PG), Isopropylmyristat (IPM), Isopropylpalmitat (IPP) Triethylcitrat (TEC), Triacetin (TRI), 1,2-Propandiol, 1,3-Propandiol, Propantiol, Pentandiol, Hexandiol, Octandiol, Decandiol (SymClariol^{®}), Dodecanol, 4-Hydroxy-Acetophenon (SymSave^{®} H), Glycerin, Butylenglykol, Pentylenglykol, Hexylenglykol, Decylenglykol, Propylencarbonat, Butylencarbonat, Glycerincarbonat, 2-Benzylheptanol, Laurylalkohol, Trimethyl-Hydroxypentyl-Isobutyrat, Glycerylcaprylat, Ethylhexylglycerin, Benzylbenzoat (BB) und insbesondere ausgewählt aus der Gruppe bestehend aus Dipropylenglykol (DPG), Diethylphtalat (DEP), Propylenglykol (PG), Isopropylmyristat (IPM), Isopropylpalmitat (IPP), Triethylcitrat (TEC), Triacetin (TRI), 1,2-Propandiol, 1,3-Propandiol, Propantiol, Pentandiol, Hexandiol, Octandiol, Decandiol (SymClariol^{®}), Dodecanol, 4-Hydroxy-Acetophenon (SymSave^{®} H), Benzylbenzoat (BB) für konkrete Anwendungen vorteilhaft ist, besonders im Hinblick auf die weiter Verarbeitung der erfindungsgemäßen Stoffmischung in einigen Endprodukten. Dementsprechend kann die erfindungsgemäße Stoffmischung mindestens zwei, drei oder vier der oben genannten Lösungsmittel enthalten.

Bezüglich der optionalen Komponente (d) hat es sich herausgestellt, dass die Kombination von mindestens zwei, vorzugsweise mindestens drei oder vier der Aroma- bzw. Duftstoffe der Gruppe (d) für konkrete Anwendungen vorteilhaft ist, besonders im Hinblick auf die weitere Verarbeitung der erfindungsgemäßen Stoffmischung in einigen Endprodukten. Dementsprechend kann die erfindungsgemäße Stoffmischung mindestens zwei, drei oder vier der der Aroma- bzw. Duftstoffe der Gruppe (d) enthalten.

In einer weiteren bevorzugten Ausführungsform enthält die erfindungsgemäße Stoffmischung einen oder mehrere Aroma- bzw. Duftstoffe der Gruppe (d), wobei diese aus der Gruppe aus 3-Phenylbutanal (Trifernal), Acetylmethylcarbinol, 2-Methyldecanal, 2-Benzyl-2-methylbut-3-ene nitrile (Citrowanil^{®}B), 3-Hexenylacetat, Styralylacetat, Rhubafuran, Anther, Prenylacetat, Styralylpropionat, Isoamylphenylacetat, beta-Damascon, Isoamylacetat, Citronellol und Cyclohexylacetat ausgewählt ist/sind.

In einer weiteren bevorzugten Ausführungsform ist die erfindungsgemäße Stoffmischung dadurch gekennzeichnet, dass sie
(i) 0,0000001 bis 80 Gew.-% Komponente (a),
(ii) 0,0000001 bis 80 Gew.-% Komponente (b),
(iii) 0,0000001 bis 98 Gew.-% Komponente (c), bevorzugt 0,0000001 bis 80 Gew.-%, besonders bevorzugt 0,0000001 bis 75 Gew.-%, und insbesondere bevorzugt 0,0000001 bis 65 Gew.-%,
(iv) 0 bis 80 Gew.-% Komponente (d),
mit der Maßgabe enthält, dass sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,00001 bis 50 Gew.-% Komponente (a),
(ii) 0,00001 bis 80 Gew.-% Komponente (b),
(iii) 0,001 bis 95 Gew.-% Komponente (c), bevorzugt 0,001 bis 80 Gew.-%, besonders bevorzugt 0,001 bis 75 Gew.-%, und insbesondere bevorzugt 0,001 bis 65 Gew.-%,
(iv) 0 bis 70 Gew.-% Komponente (d),
mit der Maßgabe enthält, dass sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,001 bis 40 Gew.-% Komponente (a),
(ii) 0,001 bis 80 Gew.-% Komponente (b),
(iii) 0,01 bis 95 Gew.-% Komponente (c), bevorzugt 0,01 bis 80 Gew.-%, besonders bevorzugt 0,01 bis 75 Gew.-%, und insbesondere bevorzugt 0,01 bis 65 Gew.-%,
(iv) 0 bis 60 Gew.-% Komponente (d),
mit der Maßgabe enthält, dass sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,1 bis 40 Gew.-% Komponente (a),
(ii) 0,1 bis 80 Gew.-% Komponente (b),
(iii) 0,1 bis 90 Gew.-% Komponente (c), bevorzugt 0,1 bis 80 Gew.-%, besonders bevorzugt 0,1 bis 75 Gew.-%, und insbesondere bevorzugt 0,1 bis 65 Gew.-%,
(iv) 0 bis 50 Gew.-% Komponente (d),
mit der Maßgabe enthält, dass sich die Mengenangaben mit gegebenenfalls weiteren Inhaltstoffen zu 100 Gew.-% addieren, bezogen auf das Gesamtgewicht der Stoffmischung.

Es hat sich herausgestellt, dass Ethanol einen positiven Einfluss nicht nur auf die erwünschte maskierende Wirkung hat, sondern auch auf die weitere Verarbeitung der erfindungsgemäße Stoffmischung in Endprodukten hat.

In diesem Sinne liegt die Menge an Ethanol in einer weiteren Ausführungsform der erfindungsgemäßen Stoffmischung ≤ 30 Gew.-%, weiter bevorzugt ≤ 20 Gew.-%, bevorzugt ≤ 15 Gew.-%, besonders bevorzugt ≤ 10 Gew.-%, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,0000001 bis 80 Gew.-% Komponente (a),
(ii) 0,0000001 bis 80 Gew.-% Komponente (b),
(iii) 0,0000001 bis 98 Gew.-% Komponente (c), bevorzugt 0,0000001 bis 80 Gew.-%, besonders bevorzugt 0,0000001 bis 75 Gew.-%, und insbesondere bevorzugt 0,0000001 bis 65 Gew.-%,
(iv) 0 bis 80 Gew.-% Komponente (d),

mit der Maßgabe enthält, dass
die Menge an Ethanol (Komponent c1)) ≤ 30 Gew.-% ist, weiter bevorzugt ≤ 20 Gew.-%, bevorzugt ≤ 15 Gew.-%, besonders bevorzugt ≤ 10 Gew.-%, bezogen auf das Gesamtgewicht der Stoffmischung,
   und
sich die Mengenangaben mit gegebenenfalls weiteren Inhaltsstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,00001 bis 50 Gew.-% Komponente (a),
(ii) 0,00001 bis 80 Gew.-% Komponente (b),
(iii) 0,001 bis 95 Gew.-% Komponente (c), bevorzugt 0,001 bis 80 Gew.-%, besonders bevorzugt 0,001 bis 75 Gew.-%, und insbesondere bevorzugt 0,001 bis 65 Gew.-%,
(iv) 0 bis 70 Gew.-% Komponente (d),

mit der Maßgabe enthält, dass
die Menge an Ethanol (Komponent c1)) ≤ 30 Gew.-% ist, weiter bevorzugt ≤ 20 Gew.-%, bevorzugt ≤ 15 Gew.-%, besonders bevorzugt ≤ 10 Gew.-%, bezogen auf das Gesamtgewicht der Stoffmischung,
   und
sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,001 bis 40 Gew.-% Komponente (a),
(ii) 0,001 bis 80 Gew.-% Komponente (b),
(iii) 0,01 bis 95 Gew.-% Komponente (c), bevorzugt 0,01 bis 80 Gew.-%, besonders bevorzugt 0,01 bis 75 Gew.-%, und insbesondere bevorzugt 0,01 bis 65 Gew.-%,
(iv) 0 bis 60 Gew.-% Komponente (d),

mit der Maßgabe enthält, dass
die Menge an Ethanol (Komponent c1)) ≤ 30 Gew.-% ist, weiter bevorzugt ≤ 20 Gew.-%, bevorzugt ≤ 15 Gew.-%, besonders bevorzugt ≤ 10 Gew.-%, bezogen auf das Gesamtgewicht der Stoffmischung,
   und
sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform ist die erfindungsgemäße Stoffmischung dadurch gekennzeichnet, dass sie
(i) 0,1 bis 40 Gew.-% Komponente (a),
(ii) 0,1 bis 80 Gew.-% Komponente (b),
(iii) 0,1 bis 90 Gew.-% Komponente (c), bevorzugt 0,1 bis 80 Gew.-%, besonders bevorzugt 0,1 bis 75 Gew.-%, und insbesondere bevorzugt 0,1 bis 65 Gew.-%,
(iv) 0 bis 50 Gew.-% Komponente (d),

mit der Maßgabe enthält, dass
die Menge an Ethanol (Komponent c1)) ≤ 30 Gew.-% ist, weiter bevorzugt ≤ 20 Gew.-%, bevorzugt ≤ 15 Gew.-%, besonders bevorzugt ≤ 10 Gew.-%, bezogen auf das Gesamtgewicht der Stoffmischung,
   und
sich die Mengenangaben mit gegebenenfalls weiteren Inhaltstoffen zu 100 Gew.-% addieren, bezogen auf das Gesamtgewicht der Stoffmischung.

In einer weiteren bevorzugten Ausführungsform der erfindungsgemäße Stoffmischung liegen die Komponenten (a) und (b) in einem Gewichtsverhältnis von etwa 100:1 bis etwa 1:100, vorzugsweise etwa 50:1 bis etwa 1:50 und insbesondere bevorzugt von etwa 1:20 bis etwa 20:1.

Eine weitere Ausführungsform der erfindungsgemäßen Stoffmischung enthält die Komponente (a+b) und (c) im Gewichtsverhältnis etwa 100:1 bis 1:100, besonders bevorzugt etwa 50:1 bis 1:50 und insbesondere bevorzugt etwa 30:1 bis 1:30.

In einer bevorzugten Ausführungsform liegt die erfindungsgemäße Stoffmischung als flüssige Mischung vor. Jedoch kann diese in eine feste Zubereitung überführt werden, z.B. durch Sprühtrocknung.

Hierin beschrieben ist ein Verfahren zur Herstellung der oben ausgeführte erfindungsgemäße Stoffmischung, umfassend die Schritte:
i) Vermischung der (a), (b), (c) und gegebenenfalls (d), wobei die Komponente (a) bis (d) in Form von Feststoffen, Lösungen, Extrakten, wässrigen Auszügen und angereichter Zubereitungen oder Konzentraten, beispielweise durch Destillation, Chromatographie oder nach dem in EP 2 075321 B1 beschrieben Verfahren, vorliegen können,
   und gegebenenfalls
ii) die so erhaltene Stoffmischung anschließend einer Sprühtrocknung unterwirft.

Im Sinne der vorliegenden Erfindung kann die erfindungsgemäße Stoffmischung mit zusätzlichen Duft- oder Aromastoffe kombiniert werden.

Hierin beschrieben ist eine Duft- oder Aromazusammensetzung, enthaltend die erfindungsgemäße Stoffmischung in Kombination mit zusätzlichen Duft- oder Aromastoffe der Gruppe (e), die in oleochemischen Zubereitungen oder Endprodukten eingearbeitet werden kann.

Bezüglich der zusätzlichen Duft- oder Aromastoffe der Gruppe (e) gelten die obenstehenden Ausführungen.

In einer bevorzugten Ausführungsform ist die Duft- oder die Aromazusammensetzung eine flüssige oder feste Aromazusammensetzung.

Hierin beschrieben ist ein Verfahren zur Herstellung der oben ausgeführten Duft oder Aromazusammensetzung, umfassend die Schritte:
i) Vermischung der (a), (b), (c) und gegebenenfalls (d), wobei die Komponente (a) bis (d) in Form von Feststoffen, Lösungen, Extrakten, wässrigen Auszügen und angereichter Zubereitungen oder Konzentraten, beispielweise durch Destillation, Chromatographie oder nach dem in EP 2 075321 B1 beschrieben Verfahren vorliegen können,
   und gegebenenfalls
ii) die so erhaltene Mischung anschließend einer Sprühtrocknung unterwirft.

Gegenstand der vorliegenden Erfindung ist die Verwendung der der oben ausgeführte Duft- oder Aromazusammensetzung zur Reduzierung und/oder Maskierung von unerwünschten Fehlnoten in oleochemischen Zubereitungen, die durch Aldehyde der Formel (III) und/oder freie Fettsäuren der Formel (IV) wie oben definiert, hervorgerufen werden.

Im Sinne der vorliegenden Erfindung lässt sich die erfindungsgemäße Stoffmischung in beliebige Produkte einsetzen, in denen es gilt, unangenehme Geruchs- oder Aromanoten, die vor allem durch Aldehyde der Formel (III) und/oder der freien Fettsäuren der Formel (IV) hervorgerufen werden, zu maskieren bzw. durch Aroma- oder Duftnoten zu ersetzen, die als angenehme Noten wahrgenommen werden. Typische Beispiele für Produktgruppen, in denen die erfindungsgemäße Stoffmischung eingesetzt werden kann, sind beispielsweise Seifenbasen, Wasch-, Spül- und Reinigungsmittel sowie kosmetische Zubereitungen. Vorzugsweise wird die erfindungsgemäße Stoffmischung in Mengen von 0,0001 bis 3 Gew.-%, bevorzugt 0,001 bis 1,5 Gew.-%, besonders bevorzugt 0,01 bis 1,2 Gew.-%, insbesondere bevorzugt 0,01 bis 1 Gew.-%, bezogen auf die Zubereitung oder Endprodukt, eingesetzt.

Hierin beschrieben sind ebenfalls Zubereitungen, insbesondere oleochemischen Zubereitungen, oder Endprodukte, die die erfindungsgemäße Stoffmischung bzw. die oben ausgeführte Duft- oder Aromazusammensetzung enthaltend die erfindungsgemäße Stoffmischung und zusätzlichen Duft- oder Aromastoffe der Gruppe (e) enthalten.

Hierin beschrieben sind Zubereitungen enthaltend
(i) die oben ausgeführten Stoffmischungen ,
   und
(ii) Aldehyde der Formel (III)
   und/oder
(iii) freie Fettsäuren der Formel (IV).

In einer bevorzugten Ausführungsform sind die Zubereitungen oleochemischen Zubereitungen.

In einer bevorzugten Ausführungsform sind die oleochemischen Zubereitungen oder Endprodukte ausgewählt aus der Gruppe bestehend aus Reinigungsmittel, Waschmittel, Lufterfrischer, Kerzen, Spülmittel, Weichspüler, Hautcreme, Lippenpflege, Make-up Entferner, Haarconditioner, Lotion, Shampoo, Duschgel und Seife und deren feste und flüssige Ausführungsformen ausgewählt, besonders bevorzugt Shampoo, Duschgel, Seife und deren feste und flüssige Ausführungsformen.

Hierin beschrieben ist zudem die Verwendung der Stoffmischung bzw. die oben ausgeführte Duft- oder Aromazusammensetzung enthaltend die Stoffmischung und zusätzlichen Duft- oder Aromastoffe der Gruppe (d) zur Reduzierung und / oder Maskierung von unerwünschten Geruchs- oder Aroma-Fehlnoten in oleochemischen Zubereitungen, insbesondere von unerwünschten Fehlnoten, die durch Aldehyde der Formel (III) und/oder freie Fettsäuren der Formel (IV) wie oben definiert, hervorgerufen werden, wobei die oleochemischen Zubereitungen aus der Gruppe bestehend aus Reinigungsmittel, Waschmittel, Lufterfrischer, Kerzen, Spülmittel, Weichspüler, Hautcreme, Lippenpflege, Make-up Entferner, Haarconditioner, Lotion, Shampoo, Duschgel und Seife und deren feste und flüssige Ausführungsformen ausgewählt sind.

Hierin beschrieben sind Zubereitungen, enthaltend die oben ausgeführten Stoffmischungen oder Duft-bzw. Aromazusammensetzungen und Aldehyde der Formel (III) und/oder freie Fettsäuren der Formel (IV).

Die Zubereitungen können den Endprodukten in Mengen von 0,0001 bis 3 Gew.-%, bevorzugt 0,001 bis 1,5 Gew.-%, besonders bevorzugt 0,01 bis 1,2 Gew.-%, insbesondere bevorzugt 0,01 bis 1 Gew.-%, bezogen auf das Endprodukt.

In einer weiteren Ausführungsform enthalten die Zubereitungen die Komponente (a) und (b), bzw. (a) und (c) oder (a) und (c+b) im Gewichtsverhältnis von 1:0,00001 bis 1:120, vorzugsweise 1:0,001 bis 1:100, insbesondere 1:0,01 bis 1:50.

Die Zubereitungen werden in Endprodukten eingesetzt, ausgewählt aus der Gruppe bestehend aus Reinigungsmittel, Waschmittel, Lufterfrischer, Kerzen, Spülmittel, Weichspüler, Hautcreme, Lippenpflege, Make-up Entferner, Haarconditioner, Lotion, Shampoo, Duschgel und Seife und deren feste und flüssige Ausführungsformen ausgewählt sind, vorzugsweise Seife und deren feste und flüssige Ausführungsformen, Shampoo und Duschgel, und besonders bevorzugt Seife und deren feste und flüssige Ausführungsformen.

Hierin beschrieben ist ebenfalls die Verwendung der Stoffmischung oder der oben ausgeführte Duft- oder Aromazusammensetzung als Fine Fragrance oder Parfüm.

### GEWERBLICHE ANWENDBARKEIT

Gemäß der vorliegenden Erfindung können Stoffmischungen, Duft- bzw. Aromazusammensetzungen und Zubereitungen hergestellt werden, die zur Reduzierung und/oder Maskierung von unerwünschten Fehlnoten in Zubereitungen, insbesondere in oleochemischen Zubereitungen, verwendet werden können.

Hierin beschrieben ist die Verwendung der oben genannten Stoffmischungen, Aromazusammensetzungen und Zubereitungen zur Herstellung von Zwischenprodukten und Endprodukten, ausgewählt aus der Gruppe bestehend aus Parfüm, Reinigungsmittel, Waschmittel, Lufterfrischer, Kerzen, Spülmittel, Weichspüler, Hautcreme, Lippenpflege, Make-up Entferner, Haarconditioner, Lotion, Shampoo, Duschgel und Seife und deren feste und flüssige Ausführungsformen ausgewählt sind, vorzugsweise Seife und deren feste und flüssige Ausführungsformen, Shampoo und Duschgel, und besonders bevorzugt Seife und deren feste und flüssige Ausführungsformen.

### BEISPIELE

Die vorliegende Erfindung wird unter Bezugnahme auf die nachstehenden Beispiele leichter zu verstehen sein. Jedoch dienen diese Beispiele lediglich zur Veranschaulichung der Erfindung und können nicht als einschränkend in Bezug auf den Schutzbereich der Erfindung ausgelegt werden.

Sofern nicht anders angegeben, beziehen sich alle Angaben auf das Gewicht.

### Beispiel 1 - Seifenstück

**Tabelle 1: Zusammensetzung der Seifenstück**

| | **Bestandteil** | **INCI** | **Seife 1 Gew.-%** | **Seife 2 Gew.-%** | **Seife 3 Gew.-%** | **Seife 4 Gew.-%** | **Seife 5 Gew.-%** | **Seife 6 Gew.-%** | **Seife 7 Gew.-%** |
|---|---|---|---|---|---|---|---|---|---|
| **Phase A** | Grundseife | Natriumtallowat, Natrium- cocoat, Natrium-Palmkern - fettsäuresalz | 97,5 | 97,5 | 97,5 | 97,5 | 97,5 | 97,5 | 97,5 |
| | Wasser | Wasser | 0,5000 | 0,5000 | 0,5000 | 0,5000 | 0,5000 | 0,5000 | 0,5000 |
| | Titandioxid | Titandioxid | 1,0000 | 1,0000 | 1,0000 | 1,0000 | 1,0000 | 1,0000 | 1,0000 |
| | | | | | | | | | |
| **Phase B** | DPG | Dipropylenglykol | 1,0000 | 0,9750 | 0,9975 | 0,9960 | 0,9685 | 0,9500 | 0,9935 |
| | gamma-Dodecalacton | | - | 0,0150 | - | - | 0,0150 | 0,0150 | - |
| | gamma-Decalacton | | - | 0,0100 | - | - | 0,0100 | 0,0100 | - |
| | Ethanol | | - | - | 0,0025 | - | 0,0025 | 0,0025 | 0,0025 |
| | Geraniol | | - | - | - | 0,0040 | 0,0040 | - | 0,0040 |
| | Limonen | | - | - | - | - | - | 0,0225 | - |

Summe 100,0000 100,0000 100,0000 100,0000 100,0000 100,0000 100,0000 Zur Herstellung der Seifenprobe 1 bis 7 wird die Phase B, die der erfindungsgemäßen Stoffmischung 1 entspricht, vorgemischt. Die Bestandteile der Phase A und die vorgemischte Phase B werden in einem Kneter vermischt. Die erhaltene Masse wird mittels eines Extruders in Stücke geformt. Anschließend wurden die Stücke für 7 Tage bei Raumtemperatur konditioniert.

Die Phasen B der Seifenstücke 1-4 und 6-7 sind nicht erfindungsgemäß, da sie die nicht alle notwendigen Komponenten (a) bis (c) gemäß der vorliegenden Erfindung enthalten. So enthält die Phase B des Seifenstückes 1 nur die Komponente (c), die Phase B des Seifenstückes 2 die Komponenten (b) und (c), die Phase B des Seifenstückes 3 die Komponente (c), die Phase B des Seifenstückes 4 die Komponente (a) und (c), die Phase B des Seifenstückes 6 die Komponente (b), (c) und (e) und das Seifenstück 7 die Komponente (a) und (c).

Die Phase B des Seifenstückes 5 ist eine erfindungsgemäße Stoffmischung, die die notwendige Komponente (a) bis (c) aufweist.

Die konditionierten Stücke wurden kodiert und sensorisch bewertet durch ein Expertenpanel von 15 Personen. Dabei wurden die wahrgenommene Gesamtintensität und die wahrgenommene Intensität der unerwünschten Fehlnoten als auch eine Beschreibung des Geruchseindrucks gemessen. Die gemessenen Werte wurden mit den Werten des Seifenmusters 1 vergleichen und die Prozent-Anteile der Fehlnoten-Intensität, auf das Seifenstück 1 bezogen, berechnet.

Die sensorischen Messungen der Seifenstücke 1 bis 7 sind in der Tabelle 2 zusammengefasst:

**Tabelle 2: Ergebnisse der sensorischen Messungen der Seifenstück 1 bis 7**

| | **Seife 1** | **Seife 2** | **Seife 3** | **Seife 4** | **Seife 5** | **Seife 6** | **Seife 7** |
|---|---|---|---|---|---|---|---|
| Gesarrtintensiät | 4,3 | 4,1 | 4,3 | 4,1 | 5,1 | 4,3 | 4,1 |
| Fehlnoten-Intensität | 4,3 | 3,0 | 4,2 | 3,3 | 0,9 | 3,3 | 3,3 |
| Reduktion der Fehlnoten-Intensität [%] im Vergleich zu Seife 1 | | 30 | 2 | 23 | 79 | 23 | 23 |
| Beschreibung | fettig, oxidiert, ranzig, stechend, kratzig | fettig, cremig, leicht oxidiert, stechend | fettig, oxidiert, ranzig, stechend, kratzig | fettig, ranzig, leicht oxidiert, blumig, kratzig | frisch, blumig, weich, cremig | fettig, cremig, oxidiert, stechend, kratzig | fettig, ranzig, leicht oxidiert, blumig, kratzig |

Weiterhin wurden die Geruchseindrücke der unterschiedlichen Seifenstück 1 bis 7 während der Phasen der Benutzung gemessen. Dazu wurden eine Hand mit dem Seifenstück 1 und die andere Hand mit einem anderen Seifenstück gewaschen. Das Expertenpanel hat dann jeweils die mit der Seife eingeschäumte Hand und dann die mit Wasser abgespülte, feuchte Hand bewertet, wobei wieder die wahrgenommene Gesamtintensität, die Fehlnoten-Intensität als auch eine Beschreibung ermittelt worden sind. Die sensorische Auswertung ist in den Tabellen 3 und 4 zusammengefasst:

**Tabelle 3: Ergebnisse der sensorischen Messungen der geschäumten Hände der Seifenstücke 1 bis 7**

| | **Seife 1** | **Seife 2** | **Seife 3** | **Seife 4** | **Seife 5** | **Seife 6** | **Seife 7** |
|---|---|---|---|---|---|---|---|
| Gesarrtintensiät | 3,5 | 3,8 | 3,6 | 3,9 | 4,3 | 3,6 | 3,9 |
| Fehlnoten-Intensität | 3,1 | 2,4 | 3,0 | 2,5 | 1,1 | 2,5 | 2,5 |
| Reduktion der Fehlnoten-Intensität [%] im Vergleich zu Seife 1 | | 23 | 3 | 19 | 65 | 19 | 19 |
| Beschreibung | kratzig, fettig, oxidiert | cremig, fettig, stechend | fettig, oxidiert, kratzig, stechend | fettig, blumig, kratzig, leicht oxidiert, | frisch, blumig, weich, cremig | fettig, cremig, oxidiert, kratzig, stechend | fettig, blumig, kratzig, leicht oxidiert, |

**Tabelle 4: Ergebnisse der sensorischen Messungen der abgespülten, feuchten Hände der Seifenstücke 1 bis 7**

| | **Seife 1** | **Seife 2** | **Seife 3** | **Seife 4** | **Seife 5** | **Seife 6** | **Seife 7** |
|---|---|---|---|---|---|---|---|
| Gesamtintensiät | 2,4 | 2,8 | 2,4 | 2,7 | 3,9 | 2,7 | 2,7 |
| Fehlnoten-Intensität | 2,1 | 1,6 | 2,0 | 1,7 | 0,8 | 1,6 | 1,6 |
| Reduktion der Fehlnoten-Intensität [%] im Vergleich zu Seife 1 | | 24 | 5 | 19 | 62 | 24 | 24 |
| Beschreibung | kratzig, muffig, oxidiert, vergoren | cremig, leicht oxidiert | oxidiert, kratzig, vergoren, leicht muffig | blumig, kratzig, leicht oxidiert | w eich, neutral, blumig, cremig | kratzig, cremig, leicht oxidiert | blumig, leicht oxidiert |

Die Ergebnisse zeigen, dass die Kombination aus Geraniol (Komponente (a)), Lactonen (Komponente (b)), und den Lösungsmitteln EtOH und DPG (Komponente (c)) im erfindungsgemäßen Seifenstück 5 deutlich niedrigere Werte bei der wahrgenommen Fehlnoten-Intensität aufweist als diese Stoffe allein. Dieser synergistische Effekt wurde bei allen Phasen der Benutzung gefunden und zeigt, dass die Stoffe länger als andere Stoffe aktiv sind. Weiterhin trifft dieser Effekt schon bei niedrigen Konzentrationen bzw. Dosierungen auf.

### Beispiel 2

Weitere Beispiele der erfindungsgemäßen Stoffmischung sind in den Tabellen 5 bis 6 zusammengefasst:

**Tabelle 5: Beispiel-Stoffmischung 2**

| **Stoff** | **Gew-%** |
|---|---|
| Dodecalacton-gamma | 30,0 |
| Decalacton-gamma | 20,0 |
| Decalacton-delta | 20,0 |
| Ethanol | 6,0 |
| 1,8-Cineol | 8,0 |
| 1,4-Cineol | 1,0 |
| Freesiol | 15,0 |
| Summe | 100,0 |

**Tabelle 6: Beispiel-Stoffmischung 3**

| **Stoff** | **Gew-%** |
|---|---|
| Dodecalacton-gamma | 20,0 |
| Decalacton-gamma | 20,0 |
| Decalacton-delta | 20,0 |
| Undecalacton-gamma | 20,0 |
| Ethanol | 4,0 |
| Geraniol | 8,0 |
| Linalool | 8,0 |
| Summe | 100,0 |

### Beispiel 3

Beispiele für Mischungen (M1) in Kombination mit weiteren Aroma- bzw. Duftstoffen als Fragrance/Parfüm oder Aroma/Flavour werden in den Tabellen 7 bis 12 zusammengefasst:

**Tabelle 7: Beispiel-Mschung 4 Fortsetzung Tabelle 7**

| **Stoff** | **Gew-%** | **Stoff** | **Gew-%** |
|---|---|---|---|
| 1,4-Cineol | 0,115 | Herbylforrriat | 3,111 |
| 2-Methyldecanal | 0,022 | Ionon-beta | 3,312 |
| 2-Phenylacetat | 0,041 | Isoamylacetate | 0,071 |
| 3-Hexenylacetat | 0,021 | Isoamylphenylacetat | 0,208 |
| 3-Methyldecalacton-gamma | 0,102 | Limonenal | 0,021 |
| Acetonphenon | 0,002 | Linalool | 1,210 |
| Agrumex HC | 3,874 | Linalylacetat | 2,978 |
| Anisaldeyhd | 0,325 | Linanylforrriat | 0,232 |
| Citronellal | 0,004 | Methylacetophenon-para | 0,002 |
| Citronellol | 0,574 | Methylanisat | 0,0330 |
| Citrow anil^{®}B | 0,223 | Methyllavenderketon | 0,038 |
| Cyclohexylaceat | 0,119 | Nectaryl | 0,018 |
| Damascon | 0,088 | Neocyclocitral | 0,011 |
| Decalacton-delta | 0,314 | Nerolidol | 0,097 |
| Decalacton-gamma | 0,455 | Nonalacton-gamma | 0,045 |
| Dihydromyrcenol | 5,987 | Octalacton-gamma | 0,025 |
| Dodecalacton-delta | 0,054 | Orangenöl/Limonen | 6,510 |
| Dodecalacton-gamma | 1,187 | Phenoxanol | 0,014 |
| DPG | 58,503 | Phenylethylalkohol | 0,748 |
| Ethanol | 1,215 | Phenylethylpropionat | 0,101 |
| Ethylphenoxyacetat | 0,002 | Rhubafuran | 0,004 |
| Ethylvanillin | 0,0001 | Stryralylpropionat | 0,021 |
| Filberton | 0,0001 | Styralylacetat | 0,190 |
| Freesiol | 0,341 | Terpinen-gamma | 1,071 |
| Geraniol | 5,100 | Undecalacton-gamma | 0,321 |
| Globalide^{®} | 0,145 | Vanillin | 0,0001 |
| Heliotropin | 0,771 | Veratraldehyd | 0,003 |
| Heptalacton-gamma | 0,013 | Vertoprenal | 0,008 |
| | | Summe | 100,0000 |

**Tabelle 8: Beispiel-Mischung 5 Fortsetzung Tabelle 8**

| **Stoff** | **Gew-%** | **Stoff** | **Gew-%** |
|---|---|---|---|
| 1,4-Cineol | 0,011 | Dodecalacton-gamma | 0,352 |
| 1,8-Cineol | 0,312 | DPG | 45,212 |
| 2-Phenylpropanal | 0,024 | Ethanol | 5,000 |
| 3-Methyldecalacton-gamma | 0,113 | Frambinon^{®} | 0,010 |
| Acetylmethylcarbinol | 0,001 | Geraniol | 3,200 |
| Agrumex HC | 2,300 | Heliotropin | 0,080 |
| Amboxid | 0,001 | Heptalacton-gamma | 0,014 |
| Amylsalicylat | 1,000 | Herbylpropionat | 4,700 |
| Anethol | 0,059 | Hydrocitronitril | 0,014 |
| Anisaldeyhd | 0,578 | lonon-beta | 4,600 |
| Anisylacetat | 0,012 | Iso ESuper | 1,000 |
| Anther | 0,112 | Isoraldein | 1,030 |
| Belanis | 0,026 | Linalool | 1,780 |
| Benzylacetat | 9,000 | Linalylacetat | 4,780 |
| Benzylsalicylat | 3,000 | Linanylformiat | 3,140 |
| Cinnamylacetat | 0,167 | Octalacton-gamma | 0,081 |
| Citronellol | 1,000 | Orangenöl/Limonen | 1,287 |
| Citrowanil^{®}B | 0,258 | Rosyrane super | 0,015 |
| Cresylmethylether-para | 0,257 | Silvial | 0,170 |
| Damascon | 0,005 | Styralylacetat | 0,198 |
| Decalacton-delta | 0,010 | Symrose^{®} | 0,020 |
| Decalacton-gamma | 0,553 | Terpinen-gamma | 0,114 |
| Dihydroeugenol | 0,089 | Trifernal | 0,010 |
| Dihydromyrcenol | 4,000 | Trimethylpyrazin | 0,0001 |
| Dodecalacton-delta | 0,100 | Undecalacton-gamma | 0,105 |
| Limonenal | 0,100 | Vanillin | 0,0001 |
| | | Summe | 100,0000 |

**Tabelle 10: Beispiel-Stoffmischung 7**

| **Stoff** | **Gew-%** |
|---|---|
| Dodecalacton-gamma | 1,5 |
| Decalacton-gamma | 1,5 |
| Anethol | 7,0 |
| Pfefferminzöl Mentha piperita Typ Willametta | 18,0 |
| Pfefferminzöl Mentha arvens is, rektifiziert | 18,0 |
| Ethanol | 1,0 |
| 1,8-Cineol | 5,0 |
| 1,4-Cineol | 2,0 |
| L-Menthol | 45,0 |
| Linalool | 1,0 |
| Summe | 100,0 |

**Tabelle 11: Beispiel-Stoffmischung 8**

| **Stoff** | **Gew-%** |
|---|---|
| Dodecalacton-gamma | 5,0 |
| Decalacton-delta | 5,0 |
| Ethanol | 2,5 |
| 1,4-Cineol | 1,0 |
| Geraniol | 0,5 |
| Linalool | 1,0 |
| Vanillin 10% in DPG | 3,0 |
| Orangenöl brasilianisch | 65,0 |
| Glycerin | 5,0 |
| Citronenöl Terpen | 12,0 |
| Summe | 100,0 |

**Tabelle 12: Beispiel-Λ/ischung 9**

| **Stoff** | **Gew-%** |
|---|---|
| 1,4-CINEOL | 0,0200 |
| DECALACTON-DELTA | 0,0030 |
| DECALACTON-GAMMA | 0,0030 |
| DODECALACTON-DELTA | 0,0100 |
| DODECANLACTON-GAMMA | 0,0100 |
| ETHANOL | 1,7500 |
| FREESIOL | 0,2000 |
| GERANIOL | 0,0040 |
| ROSMARINÖL | 0,5000 |
| DIHYDROMYRCENOL | 11,0000 |
| HERBAFLORAT (ESSIGSAEURETRlCYCLO[5.2.1.0]-4-DECEN-8-YLESTER) | 5,0000 |
| HEXYLZIMTALDEHYD ALPHA | 10,0000 |
| CUMARIN | 1,0000 |
| DIPHENY LOXID | 5,0000 |
| LILIAL^{®} (PROPANAL, 2-METHYL-3-(4-TERT.-BUTYLAHENYL)-) | 4,0000 |
| GALAXOLID^{®} 50% in DEP (1,1,2,3,3,8-Hexamethyl-1,2,3,5,7,8-hexahydro-6-oxa-cyclopenta[b]naphthalen) | 12,0000 |
| ISORALDEIN^{®} 70 ((E)-3-Methyl-4-(2,6,6-trimethyl-cyclohex-2-enyl)-but-3-en-2-on) | 3,0000 |
| ALLYLAMYLGLYCOLAT | 0,7000 |
| ISO E SUPER^{®} (BICYCLO[4.4.0]DECEN, 3-ACETYL-3,4,10,10-TETRAMETHYL-1(6)-) | 8,0000 |
| ROSEN BASE | 8,0000 |
| GALBANUM BASE | 8,0000 |
| APFEL BASE | 2,5000 |
| OZONIL 10% in DPG (DODECENYLCYANID, 1Z-) | 1,5000 |
| MELONAL^{®} 10% in DPG (HEPTENAL, 2,6-DIMETHYL-5-) | 0,3000 |
| METHYLOCTINCARBONAT 10% in DPG | 0,5000 |
| DAMASCON DELTA 10% in DPG (1-(2,6,6-Trimethyl-cyclohex-3-enyl)-but-2-en-1-one) | 0,7000 |
| SANDRANOL^{®} (BUTENOL, 2-ETHYL-4-(2,2,3-TRIMETHYL-3-CYCLOPENTENYL)-2E-) | 2,0000 |
| VERTOCITRAL (CYCLOHEXEN, TRANS-2,4-DIMETHYL-1-FORMYL-3-) | 0,3000 |
| AGRUMEX (ESSIGSAEURE-2-TERT.-BUTYLCYCLOHEXYLESTER) | 8,0000 |
| BENZYLACETON | 1,0000 |
| ALDEHYD C12 MNA (Methylnonylacetaldehyd) | 0,5000 |
| PROJASMON P (CYCLOPENTANON, 2-HEPTYL-) | 1,0000 |
| NEROLIN YARA YARA | 1,5000 |
| INTRELEVENALDEHYD (10-Undecenal) 10% in DPG | 0,5000 |
| PATCHOULIÖL | 1,5000 |
| Summe | 100,0000 |

**Tabelle 13: Beispiel-Λ/ischung 10**

| **Stoff** | **Gew-%** |
|---|---|
| 1,4-CINEOL | 0,3000 |
| DECALACTON-DELTA | 0,0100 |
| DODECANLACTON-GAMMA | 0,4000 |
| ETHANOL | 5,0000 |
| GERANIOL | 0,2000 |
| DPG | 32,2800 |
| CEDERNHOLZÖL | 0,8000 |
| AMBROCENIDE^{®} 10% in DPG | 0,1000 |
| ROSMARINÖL | 0,8000 |
| DIHYDROMYRCENOL | 8,0000 |
| ISOBUTYLCHINOLIN | 0,0500 |
| AMBROXAN | 0,1000 |
| LIGUSTRAL (CYCLOHEXEN, TRANS-2,4-DIMETHYL-1-FORMYL-3-) | 0,2000 |
| AMYLSALICYLAT N/ISO | 2,4000 |
| CITRONELLOL | 0,8000 |
| VERTOFIX (1-(3,6,8,8-Tetramethyl-2,3,4,7,8,8a-hexahydro-1H-3a,7-methano-azulen-5-yl)-ethanone) | 3,0000 |
| HEXYLZIMTALDEHYDALPHA | 5,0000 |
| CUMARIN | 0,4000 |
| COUMARONE (BENZOFURAN, 2-ACETYL-) | 0,0600 |
| ISOBORNY LACETAT | 3,0000 |
| KAMPFER | 0,8000 |
| LILIAL^{®} (PROPANAL, 2-METHYL-3-(4-TERT.-BUTYLAHENYL)-) | 3,0000 |
| LINALOOL | 4,0000 |
| LINALYLACETAT | 4,0000 |
| TERPINEOL | 10,0000 |
| GALAXOLID^{®} 50% in DEP (1,1,2,3,3,8-Hexamethyl-1,2,3,5,7,8-hexahydro-6-oxa-cyclopenta[b]naphthalin) | 2,4000 |
| GLOBALIDE^{®} (PENTADECEN-1,15-OLID, 11E/Z) | 0,5000 |
| ETHYLENBRASSYLAT (BRASSYLSÄUREETHANDIOLESTER) | 2,4000 |
| AGRUNITRIL (HEPTENYLCYANID, 2,6-DIMETHYL-5-) | 1,6000 |
| ISORALDEIN^{®} 70 (E)-3-Methyl-4-(2,6,6-trimethyl-cyclohex-2-enyl)-but-3-en-2-on) | 2,0000 |
| ALLYLAMYLGLYCOLAT (ESSIGSÄUREALLYLESTER, 2-METHYLBUTOXY-) | 1,5000 |
| DAMASCONE DELTA ((1-(2,6,6-Trimethyl-cyclohex-3-enyl)-but-2-en-1-on) | 0,2000 |
| TIMBEROL^{®} (CYCLOHEXAN, 2,2,6-TRIMETHYL-1-(3-HYDROXYHEXYL)-) | 0,2000 |
| CITRONENÖL TERPENE | 2,0000 |
| CALONE^{®} 1951 10% in DPG (BENZODIOXEPINON, 7-METHYL-3,4-DIHYDRO-3-) | 0,4000 |
| EUGENOL | 0,1500 |
| FARENAL^{®} (UNDECENAL, 2,6,10-TRIMETHYL-9-) | 0,0500 |
| LIMETTENÖL | 1,2000 |
| ANETHOL | 0,4000 |
| METHYLNAPHTHYLKETON BETA | 0,3000 |
| Summe | 100,0000 |

**Tabelle 14: Beispiel-Λ/ischung 11**

| **Stoff** | **Gew-%** |
|---|---|
| 1,4-CINEOL | 0,3000 |
| HEPTALACTON-GAMMA | 0,0020 |
| OCTALACTON-GAMMA | 0,0020 |
| NONALACTON-GAMMA | 0,0020 |
| UNDECALACTON-GAMMA | 0,0020 |
| DECALACTON-DELTA | 0,0020 |
| DODECANLACTON-GAMMA | 0,4000 |
| ETHANOL | 2,0000 |
| FREESIOL | 1,5000 |
| DPG | 2,6400 |
| JASMOL | 0,5000 |
| TOCOPHEROL | 0,0500 |
| CASSIS BASE | 1,0000 |
| NELKENBLÜTENÖL | 0,5000 |
| PATCHOULIÖL | 0,5000 |
| DIHY DROMY RCENOL | 6,0000 |
| HEXYLSALICYLAT | 2,0000 |
| HEDION^{®} (METHYL-CIS/TRANS-DIHYDROJASMONAT) | 21,0000 |
| ORANGENÖL | 2,5000 |
| GLOBALIDE^{®} (PENTADECEN-1,15-OLID, 11E/Z) | 2,0000 |
| CYCLOPENTENYL)-4-) | 0,5000 |
| LAVANDINÖL GROSSO | 2,0000 |
| YSAMBER^{®} K (ISOLONGIFOLANONETHANDIOLKETAL) | 2,0000 |
| HEXYLZIMTALDEHYDALPHA | 8,0000 |
| LILIAL^{®} (PROPANAL, 2-METHYL-3-(4-TERT.-BUTYLPHENYL)-) | 1,0000 |
| LINALOOL | 6,0000 |
| LINALYLACETAT | 5,0000 |
| TERPINEOL | 1,8000 |
| ETHYLENBRASSYLAT (BRASSYLSÄUREETHANDIOLESTER) | 2,0000 |
| ALLYLAMYLGLYCOLAT (ESSIGSÄUREALLYLESTER, 2-METHYLBUTOXY-) | 1,0000 |
| ISO E SUPER^{®} (BICYCLO[4.4.0]DECEN, 3-ACETYL-3,4,10,10-TETRAMETHYL-1(6)-) | 4,5000 |
| KEPHALIS (CYCLOHEXANON, 3,3,5,5-TETRAMETHYL-4-(1-ETHOXYVINYL)-) | 4,5000 |
| BERGAMOTTE BASE | 8,0000 |
| FLORALOZONE (PROPANAL, 2-METHYL-2-(4-ETHYLBENZYL)- | 1,0000 |
| MANDARNENALDEHY D 10% in TEC (DODECENAL, 2E-) | 0,5000 |
| LIGUSTRAL^{®} 10% in DPG (CYCLOHEXEN, TRANS-2,4-DIMETHYL-1-FORMYL-3-) | 0,8000 |
| DAMASCON ALPHA 1% in DPG ((E/Z)-1-(2,6,6-Trimethyl-cyclohex-2-enyl)-but-2-en-1-on) | 2,0000 |
| FARENAL^{®} 1% in DPG (UNDECENAL, 2,6,10-TRIMEHYL-9-) | 1,5000 |
| LEAFOVERT^{®} 10% in DPG (KOHLENSAEURE-3Z-HEXENYLMETHYLESTER) | 2,0000 |
| CALONE^{®} 1951 10% in DPG (BENZODIOXEPINON, 7-METHYL-3,4-DIHYDRO-3-) | 3,0000 |
| Summe | 100,0000 |

**Tabelle 15: Beispiel-Λ/ischung 12**

| **Stoff** | **Gew-%** |
|---|---|
| 1,4-CINEOL | 0,3000 |
| HEPTALACTON-GAMMA | 0,0020 |
| OCTALACTON-GAMMA | 0,0020 |
| NONALACTON-GAMMA | 0,0020 |
| UNDECALACTON-GAMMA | 0,0020 |
| DECALA CTON- DELTA | 0,0020 |
| DODECANLACTON-GAMMA | 0,4000 |
| ETHANOL | 2,0000 |
| FREESIOL | 1,5000 |
| DPG | 1,7400 |
| TOCOPHEROL | 0,0500 |
| NELKENBLÜTENÖL | 1,0000 |
| PATCHOULIÖL | 5,0000 |
| DIHY DROMY RCENOL | 6,0000 |
| ISORALDEIN^{®} ((E)-3-Methyl-4-(2,6,6-trimethyl-cyclohex-2-enyl)-but-3-en-2-on) | 2,0000 |
| EBANOL (PENTEN-2-ON, 3-METHYL-5-(2,2,3-TRIMETHYL-3-CYCLOPENTENYL)-4E/Z-) | 1,5000 |
| GLOBALIDE^{®} (PENTADECEN-1,15-OLID, 11E/Z) | 6,0000 |
| HEXYLZIMTALDEHYD ALPHA | 4,0000 |
| CUMARIN | 2,0000 |
| LILIAL^{®} (PROPANAL, 2-METHYL-3-(4-TERT.-BUTYLAHENYL)-) | 3,0000 |
| LINALOOL | 2,0000 |
| LINALYLACETAT | 3,0000 |
| VANILLIN | 2,0000 |
| LYRAL^{®} (CYCLOHEXEN, 4-FORMYL-2-(4-HYDROXY-4-METHYL-PENTYL)-) | 4,0000 |
| HEDION^{®} (METHYL-CIS/TRANS-DIHYDROJASMONAT) | 3,0000 |
| EVERNYL^{®} (BENZOESAEUREMETHYLESTER, 2,4-DIHYDROXY-3,6-DIMETHYL-) | 0,5000 |
| CEDRAMBER (CEDRYLMETHYLETHER) | 2,0000 |
| ISO E SUPER^{®} (BICYCLO[4.4.0]DECEN, 3-ACETYL-3,4,10,10-TETRAMETHYL-1(6)-) | 15,0000 |
| GERANIUM BASE | 2,0000 |
| BERGAMOTTE BASE | 7,0000 |
| BEIFFUSSÖL | 1,0000 |
| GALBANUMÖL 10% in DPG | 1,5000 |
| AMBROCENIDE^{®} 0,1% in DPG | 2,0000 |
| CYCLOGALBANAT^{®} 10% in DPG (CYCLOHEXYLOXYESSIGSÄUREALLYLESTER) | 0,5000 |
| CISTUSÖL 10% in DPG | 1,0000 |
| KRAUSEMINZÖL (SPEARIMINT) 10% in DPG | 1,0000 |
| AURELIONE (CYCLOHEXADECENON, 7/8E/Z-) | 12,0000 |
| AMBROXID | 0,5000 |
| MANDARINENÖL | 0,5000 |
| LAVANDINÖL GROSSO | 3,0000 |
| Summe | 100,0000 |

**Tabelle 16: Beispiel-Λ/ischung 13**

| **Stoff** | **Gew-%** |
|---|---|
| DECALACTON-DELTA | 0,0020 |
| DODECANLACTON-GAMMA | 0,4000 |
| ETHANOL | 2,0000 |
| GERANIOL | 1,5000 |
| DPG | 2,8480 |
| TOCOPHEROL | 0,0500 |
| AGRUMEX LC | 0,5000 |
| ALDEHYDE C14 SOGENANNT | 0,2000 |
| AMAROCIT^{®} | 0,2000 |
| AMBERWOOD^{®} F | 1,0000 |
| BENZOIN SIAM RESIN 50% IN BB | 0,3000 |
| BERGAMOT SYNTHESSENCE AFRIKANISCH | 4,0000 |
| ZEDERNHOLZÖL VIRGINIA | 3,0000 |
| CEDRAMBER | 1,5000 |
| COUMARIN | 0,3000 |
| DIETHYLMALONAT | 0,8000 |
| DIHY DROMY RCENOL | 0,8000 |
| ETHYLDECADIENOAT TRANS CIS-2,4 10% in IPM | 1,0000 |
| ETHYL VANILLIN | 0,4000 |
| ETHYLENBRASSYLAT | 12,0000 |
| FLOROSA | 3,0000 |
| GERANYLACETAT | 0,5000 |
| INGWERÖL CHINESISCH | 0,2000 |
| GIVESCONE 10% in DPG | 0,6000 |
| HEDIONE | 6,5000 |
| HELIONAL | 3,0000 |
| HEXENYLACETATCIS-3 10% in DPG | 2,0000 |
| HEXYL SALICYLAT | 6,5000 |
| INDOLE 10% in DPG | 0,3000 |
| ISO E SUPER | 22,5000 |
| LINALOOL | 3,0000 |
| LINALYLACETAT | 4,0000 |
| METHYLANTHRANILAT | 0,2000 |
| METHYL NAPHTYL KETON BETA KRIST. | 0,3000 |
| NEROLIDOL | 0,5000 |
| ORANGENÖL BRASILIANISCH | 6,0000 |
| PRENYLACETAT 10% in DPG | 1,0000 |
| SANDRANOL^{®} | 1,0000 |
| STYRALLYLACETAT 10% in DPG | 0,8000 |
| SYMROXANE^{®} | 0,3000 |
| TETRAHYDROLINALOOL | 5,0000 |
| Summe | 100,0000 |

Im Folgenden wird die vorliegende Erfindung anhand ausgewählter, konkreter Anwendungsbeispiele näher beschrieben. Wie bereits hingewiesen, dienen diese Beispiele nur zur Verdeutlichung der Erfindung, ohne diese einzuschränken bzw. darauf zu beschränken.

### Flüssigseife

**Tabelle 17: Flüssigseife**

| | **Bestandteil** | **INCI** | **Gew.-%** |
|---|---|---|---|
| **Phase A** | Wasser | Water | 77,4360 |
| | Rantacare PS 10 | Sodium Laureth sulfate, Lauryl Glucoside | 20,0000 |
| | Preservative | | 0,5000 |
| | Natrium Chlorid | Sodium Chlorid | 1,4000 |
| | Citronensäure Monohydrat | Citric Acid | 0,1640 |
| | | | |
| **Phase B** | DPG | Dipropylenglykol | 0,4253 |
| | gamma-Dodecalacton | | 0,0122 |
| | gamma-Decalacton | | 0,0090 |
| | Ethanol | | 0,0500 |
| | Geraniol | | 0,0025 |
| | Linalool | | 0,0010 |
| | | Summe | 100,0000 |

Zur Herstellung der Phase A werden alle aufgeführten Bestandteile gemäß der Reihenfolge dieser Liste, nacheinander, bei Raumtemperatur und unter Rühren zusammengefügt. Die Phase B, die der erfindungsgemäßen Stoffmischung 1 entspricht, wird vorgemischt und zur fertigen Phase A hinzugefügt. Falls erforderlich, kann der pH-Wert mit Citronensäure oder Natriumhydroxid auf 6,0 eingestellt werden.

### Shampoo

**Tabelle 18: Shampoo**

| **Bestandteil** | **INCI** | **Gew.-%** |
|---|---|---|
| Wasser | Water | 71,3000 |
| Citronensäure Monohydrat | Citric acid | 0,1000 |
| Euperlan PK 771 | Glycol Distearate, Sodium Laureth Sulfate, Cocamide MEA, Laureth-10 | 6,0000 |
| Natrium Chlorid | Sodium Chlorid | 1,4000 |
| Rantacare PS 10 | Sodium Laureth Sulfate, Lauryl Glucoside | 20,0000 |
| Preservativ | | 0,5000 |
| Beispiel-Mischung 4 | Parfum | 0,7000 |
| | Summe | 100,0000 |

Zur Herstellung werden alle aufgeführten Bestandteile gemäß der Reihenfolge dieser Liste, nacheinander, bei Raumtemperatur und unter Rühren zusammengefügt. Falls erforderlich, kann der pH-Wert mit Citronensäure oder Natriumhydroxid auf 6,0 eingestellt werden.

### Waschseife

**Tabelle 19: Waschseife**

| **Bestandteil** | **INCI** | **Gew.-%** |
|---|---|---|
| Britesil H 20 | Sodium Silicate | 14,6500 |
| Dolomite | Sudesan | 25,0000 |
| Grundseife HTS | SodiumTallowate, Sodium Cocoate, sodium | |
| | Palm Kernelate | 45,0000 |
| Marlon ARL | C-10-13, ABS-Na, Pulver | 6,7000 |
| Texapon Z HK Pulver | Sodium fatty alcohol C12-C18 | 6,6000 |
| Titanium Dioxid | Titanium Dioxide | 1,0000 |
| Kosmfst. Säureblau 7, C.I.42080 Pulver | | 0,2000 |
| Beispiel-Mischung 4 | Parfum | 0,8500 |
| | Summe | 100,0000 |

Die Bestandteile werden in einem Kneter vermischt und mittels eines Extruders in ein Seifenstück geformt.

### Make-up Entferner

Die Herstellung erfolgt in einem Becomix.

Zur Herstellung der Phase A wird das Glycerin und das Surfhope^{®} C1216 in den Becomix gegeben und bei -0,5 bar mit 0,3 m/s gerührt, danach mittels Turrax bei 3 m/s für 4 Minuten homogenisiert. Wenn das Surfhope^{®} C1216 vollständig gelöst ist, wird das Surfhope^{®} C1616 hinzugefügt, wobei das Rühren bei 0,3m/s fortgesetzt wird.

Die anschließende Homogenisierung erfolgt bei 3m/s für 4 Minuten mittels Turrax. Danach wird das Wasser hinzugefügt und erneut homogenisiert mittels Turrax mit 3m/s für 6 Minuten.

Dieses Gemisch wird unter Rühren bei 0,3m/s auf 80 °C erhitzt, dann auf 50 °C gebracht und wiederum homogenisiert mittels Turrax mit 3m/s für 2 Minuten. Anschließend wird die Temperatur auf 80 °C erhöht und ein Druck von -0,6 bar eingestellt.

**Tabelle 20: Make-up Entferner**

| **Bestandteil** | **INCI** | **Gew.-%** |
|---|---|---|
| **Phase A** | | |
| Glycerin | Glycerin | 10,0000 |
| Surfhope^{®} C-1216 | Sucrose Laurate | 3,0000 |
| Surfhope^{®} C-1616 | Sucrose Palmitate | 3,0000 |
| Wasser | Water (Aqua) | 5,0000 |
| **Phase B** | | |
| Neutral oil | Caprylic/Capric Triglyceride | 47,7100 |
| Dragoxat^{®} 89 | Ethylhexyl Isononanoate | 30,0000 |
| Ionol CP | BHT | 0,0500 |
| **Phase C** | | |
| Symdiol^{®} 68 | 1,2-Hexanediol, Caprylyl Glycol | 0,8000 |
| **Phase D** | | |
| Beispiel-Mischung 2 | Parfum | 0,1500 |
| Colour I | Colour | 0,1500 |
| Colour II | Colour | 0,1400 |
| | Summe | 100,0000 |

Die Herstellung der Phase B erfolgt bei 80 °C. Bei einer Temperatur von 80 °C werden die Bestandteile der Phase B sehr langsam nacheinander und sehr langsam vermischt und dann bei 0,5 m/s gerührt sowie mittel Turrax bei 3m/s für 2 Minuten homogenisiert. Wenn ein Viertel des Öls in den Kessel gegeben worden sind, erfolgt ein Homogenisierungsschritt mittels Turrax mit 5 m/s für 4 Minuten.

Nachdem die Hälfte der Mange an Öl hinzugefügt worden sind, wird erneut homogenisiert mittels Turrax mit 5 m/s für 4 Minuten sowie die Rührgeschwindigkeit auf 0,8 m/s erhöht.

Wenn drei Viertel des Öls hinzugefügt wurden, schließt sich ein Homogenisierungsschritt mittels Turrax mit 5 m/s für 4 Minuten und eine Erhöhung der Rührgeschwindigkeit auf 1 m/s. Dann wird die restliche Menge des Öls hinzugegeben und mit 1,2 m/s gerührt.

Nach dem Hinzufügen der Phase C, wird der Druck auf -0,8 bar erhöht und mittels Turrax bei 5 m/s für 4 Minuten homogenisiert.

Die Zubereitung wird unter Rühren mit 0,5 m/s auf 25 °C abgekühlt, wobei die Rührgeschwindigkeit dabei reduziert. Dann wird die Phase D bei 40 °C hinzugegeben.

Colour I: 0,1 %ige Lösung in Isopropylpalmitat von D&C Red N °17 C.I. 26100 Colour II: 0,1 %ige Lösung in Isopropylpalmitat von Phat Brown DC 8206: C.I. 47000, C.I. 26100, C.I. 60725

Die finale Zubereitung hat eine Viskosität von 69280 cP.

### Spülmittel

**Tabelle 21: Spülmittel**

| **Bestandteil** | **INCI** | **Gew.-%** |
|---|---|---|
| Wasser, demineralisiert | Water | 41,8000 |
| Glucopon 650 EC | Coco Glucoside | 4,0000 |
| Zetesol NL-2 | Sodium Laureth Sulfate | 45,0000 |
| Dehyton AB 30 | Coco-Betaine | 8,0000 |
| Ethanol 96% | Ethanol | 1,0000 |
| Parmatol MBX | Benisothiazolinone, Methylisothiazolinone, | 0,1000 |
| | Laurylarrine, Dipropylenediarrine | |
| Beispiel-Mischung 4 | Parfum | 0,1000 |
| | Summe | 100,0000 |

Zur Herstellung werden alle Bestandteile in der Reihenfolge der Liste hinzugefügt, wobei zu beachten ist, dass jeder Bestandteil vollständig gelöst ist, bevor der nächste Bestandteil hinzugegeben wird. Der pH-Wert wird mittels Citronensäure auf pH = 7 eingestellt.

### Haarconditioner

**Tabelle 22: Haarconditioner**

| **Bestandteil** | **INCI** | **Gew.-%** |
|---|---|---|
| **Phase A** | | |
| Hostaphat Kw 340 D | Trieteareth-4 Phospate | 10,0000 |
| Medium Mineral Oil | Paraffinium Liquidum | 6,0000 |
| Lunacera M | Ozocerite | 5,0000 |
| Isopropylmyristat | Isopropyl yristate | 5,0000 |
| Xiameter^{®} PMX-200 Silicone Fluid | Dimethicone | 0,5000 |
| Lanolin Alcohol | Lanolin Alcohol | 0,5000 |

| **Phase B** | | |
|---|---|---|
| Wasser | Water (Aqua) | 29,1000 |
| Preservative | | 0,8000 |
| Glycerin, 99,5 P. | Glycerin | 12,0000 |
| Citronensäure 20% wässrige Lösung | Citric acid | 1,3000 |

| **Phase C** | | |
|---|---|---|
| Wasser | Water (Aqua) | 28,9000 |
| Carbopol^{®} Ultrez-10 | Caromer | 0,2000 |
| Triethanolarrin 99% | Triethanolamine | 0,3000 |

| **Phase D** | | |
|---|---|---|
| Beispiel-Mischung 5 | Parfum | 0,4000 |
| | Summe | 100,0000 |

Zur Herstellung des Haarconditioners werden alle Bestandteile der Phase A vermischt und auf 75 °C erwärmt.

Zur Herstellung der Phase B wird zunächst das Wasser auf 80 °C erwärmt und dann die restlichen Bestandteile der Phase B hinzugemischt und homogenisiert. Dann wird die Phase A der Phase B unter Rühren hinzugefügt.

Zur Herstellung der Phase C wird das Ultrez-10 im Wasser unter starkem Rühren quellen gelassen, dann das Triethanolamin hinzugeben, um eine klare Gelzubereitung zu erhalten, die dann auf 45 °C erwärmt wird. Zu der 45 °C warmen Phase C werden das Gemisch aus Phase A und B hinzugefügt und bei Raumtemperatur homogenisiert.

Dann wird die Phase D die Beispiel-Mischung 5 ergänzt.

Falls erforderlich, sollte der pH-Wert mittels Citronensäure oder Natriumhydroxid auf pH = 4 eingestellt werden.

### Flüssigvollwaschmittel

**Tabelle 23: Flüssigvollwaschmittel**

| **Bestandteil** | | **INCI** | **Gew.-%** |
|---|---|---|---|
| 1 | Wasser, demineralisiert | Water | 39,7000 |
| 2 | Tinopal CBS-X | Disodium Distyrylbiphenyl Disulfonate | 0,1000 |
| 3 | Edenor K 12-18 | Cocos fatty acids | 7,5000 |
| 4 | Kalium Hydroxid 50% Lösung | Potassium Hydroxide | 4,3000 |
| 5 | Propylenglykol 1,2 | Propan-1,2-diol | 5,0000 |
| 6 | Hoesch T 9 90% | Trideceth-9 | 12,0000 |
| 7 | Parmatol A 26 | Mxture of 5-Chloro-2-methyl-2H-isothiazol-3-one and 2-Methyl-2H-isothiazol-3-one | 0,2000 |
| 8 | Hoesch NAS 60 | Sulfonic acid, C 13-17-sec-Alkyl-, Sodium salt | 17,0000 |
| 9 | Triethanolamin pure C | Triethanolamine | 2,0000 |
| 10 | Trinatriumcitrat Dihydrat | Tri-sodiumcitrate-dihydrate | 5,0000 |
| 11 | Hoesch Phos DET 32 D | Diethylenetriamine Pentamethylene Phosphonic acid Sodium salt | 3,0000 |
| 12 | Ethanol 96% | Ethanol | 3,0000 |
| 13 | Alcalase 2,5 L | Protease | 0,4000 |
| 14 | Termamyl 300L | Alpha-Amylase | 0,3000 |
| 15 | Beispiel-Mschung 5 | Parfum | 0,5000 |
| | | Summe | 100,0000 |

Zur Herstellung werden die Bestandteile 1 und 2 vermischt und auf 50 °C erwärmt. Dann werden die Bestandteile 3 und 4 dazugegeben und gerührt bis sich Seife gebildet hat. Nach dem die Bestandteile 5 bis 10 in der Reihenfolge der Liste hinzugefügt und auf 30 °C abgekühlt wurde, werden die Bestandteile 11 bis 14 in der Reihenfolge der Liste hinzugegeben. Falls es erforderlich ist, wird der pH-Wert auf 8,5 eingestellt.

### Flüssigwaschmittel

Zur Herstellung wird in einem Gefäß das Wasser vorgelegt und unter Rühren auf 70 °C erwärmt. Dann werden die Bestandteile 2 und 3 in dieser Reihenfolge hinzugefügt und für 30 Minuten ohne weiteres Erwärmen gerührt. Dann werden alle Bestandteile in der Reihenfolge der Liste hinzugefügt, wobei zu beachten ist, dass jeder Bestandteil vollständig gelöst ist, bevor der nächste Bestandteil hinzugegeben wird. Der pH-Wert wird mittels Citronensäure auf pH = 9 eingestellt.

**Tabelle 24: Flüssigwaschmittel**

| **Bestandteil** | | **INCI** | **Gew.-%** |
|---|---|---|---|
| 1 | Wasser, demineralisiert | Water | 48,3000 |
| 2 | Edenor K 12-18 | Cocos fatty acids | 3,0000 |
| 3 | Natrium Hydroxid 50% Lösung | Sodium Hydroxide | 2,5000 |
| 4 | Novethix L-10 Polymer | NRC Nordmann, Rassmann | 2,5000 |
| 5 | Zetesol NL-2 | Sodium Laureth Sulfate | 30,0000 |
| 6 | Hoesch AE50 | sodium Dodecylbenzenesulfonate | 3,0000 |
| 7 | Dehydol LT 7 | | 3,0000 |
| 8 | Hoesch Phos DET 32 D | Diethylenetriamine Pentamethylene Phosphonic acid Sodium salt | 1,0000 |
| 9 | Ethanol 96% | Ethanol | 2,0000 |
| 10 | Propylenglykol 1,2 | Propan-1,2-diol | 2,0000 |
| 11 | Trinatriumcitrat Dihydrat | Tri-sodiumcitrate-dihydrate | 2,0000 |
| 12 | Beispiel-Mischung 5 | Parfum | 0,5000 |
| 13 | Parmatol A 26 | Mixture of 5-Chloro-2-methyl-2H-isothiazol-3-one and 2-Methyl-2H-isothiazol-3-one | 0,2000 |
| | | Summe | 100,0000 |

### Weichspüler

**Tabelle 25: Weichspüler**

| **Bestandteil** | | **INCI** | **Gew.-%** |
|---|---|---|---|
| 1 | Wasser, demineralisiert | Water | 93,1000 |
| 2 | Parmetol K 40 | N, S-Heterocyclen | 0,1000 |
| 3 | Xiameter AFE-1520 | Simethicone | 0,3000 |
| 4 | Rew oquat WE 18 | Di-(Talg Carboxyethyl) Hydroxyethyl methylammoniummethosulfat | 5,5000 |
| 5 | Beispiel-Mischung 5 | Parfum | 1,0000 |
| | | Summe | 100,0000 |

Zur Herstellung wird in einem Gefäß das Wasser vorgelegt und auf 45 °C erwärmt. Dann wird unter starkem Rühren der Bestandteil 2 hinzugefügt und kontinuierlich bis auf 50 °C erwärmt bis der Bestandteil 2 vollständig gelöst ist. Dann werden nacheinander separat die Bestandteile 3 und 4 in der Reihenfolge der Liste hinzugefügt. Nach dem Abkühlen auf 25 °C wird der Bestandteil 5 ergänzt und dann für 30 Minuten stark gerührt. Zum Schluss wird der pH-Wert mit Citronensäure oder Natriumhydroxid auf pH = 3 eingestellt.

### Lippenpflegestift

Zur Herstellung werden alle Bestandteile in der Reihenfolge der Liste ohne da Parfüm geschmolzen und während des Erwärmens auf 70 °C homogenisiert. Bei einer Temperatur von 50 °C wird das Parfüm hinzugefügt und homogenisiert.

**Tabelle 26: Lippenpflegestift**

| **Bestandteil** | **INCI** | **Gew.-%** |
|---|---|---|
| Cetiol 868 | Ethylhexyl Stearate | 5,0000 |
| Cutina^{®} FS 45 | Stearic acid, Palrritic acid | 1,5000 |
| Elfacos E200 | Methoxy PEG-22 and Dodecyl Glycol Copolymer | 4,0000 |
| Ivarlan 3360 | Glyceryl Lanolate | 3,0000 |
| Jojoba oil | | 1,0000 |
| Medium rrineral oil | Paraffinium Liquidum | 25,9950 |
| Neutral oil | Caprylic/Capric Triglyceride | 10,0000 |
| Paraffin 52-54 | Paraffin | 12,0000 |
| Permulgin 3220 | Ozokerite | 20,0000 |
| Permulgin 3430 | Copernicia Cerifera (Carnauba) Wax | 2,0000 |
| Prisorine Sqs 3758 | Hydrogenated Polyisobutene | 5,0000 |
| Softisan 100 | Hydrogenated Cocoglycerides | 5,0000 |
| Super Hartolan | Lanolin Alcohol | 1,4000 |
| Titaniumdioxid | Titanium Dioxide | 0,1000 |
| Xiameter^{®} PMX-0245 Cyclosiloxane | Cyclopentasiloxane | 4,0000 |
| Beispiel-Mischung 3 | Parfum | 0,0050 |
| | Summe | 100,000 |

### Nachtcreme

**Tabelle 27: Nachtcreme**

| **Bestandteil** | **INCI** | **Gew.-%** |
|---|---|---|
| **Phase A** | | |
| Dragosan W/O P | Sorbitan Isostearate, Hydrogenated Castor oil, Beewax (Cera Alba) | 5,0000 |
| Dragoxat^{®} 89 | Ethylhexyl Isononanoate | 5,0000 |
| Medium Mineral Oil | Paraffinium Liquidum | 15,1000 |
| Tece Ozokerit N 325/II | Ozokerite | 2,3000 |
| Vaseline | Petrolatum | 4,5000 |

| **Phase B** | | |
|---|---|---|
| Wasser | Water (Aqua) | 64,0970 |
| Glycerin, 99,5 P. | Glycerin | 3,0000 |
| Magnesiumsulfat-Heptahydrat | Magnesium Sulfate | 0,7000 |
| Preservative Complex (ND Liquid) | Triethylene Glycol, Irridazolidinyl Urea, Methylparaben, | 0,3000 |

| **Phase C** | | |
|---|---|---|
| Beispiel-Mischung 2 | Parfum | 0,0030 |
| | Summe | 100,0000 |

Zur Herstellung werden jeweils die Phase A und B einzeln auf ca. 80 °C erwärmt. Dann wird die Phase B der Phase A hinzugefügt unter Verwendung eines Ultra-Turrax und emulgiert. Unter Rühren mit einem Schaufelrührer wird dieses Gemisch abgekühlt und dann erneut bei ca. 60 °C emulgiert. Wenn die Mischung eine Temperatur von ca. 35 °C erreicht hat, wird die Phase C hinzugefügt.

### Body Lotion

**Tabelle 28: Bodylotion**

| **Bestandteil** | **INCI** | **Gew.-%** |
|---|---|---|
| **Phase A** | | |
| Covabsorb | Ethylhexyl Methoxycinnamate, Butyl | 0,4000 |
| Dragoxat^{®} 89 | Ethylhexyl Isononanoate | 3,7500 |
| Emulium 22 | Tribehenin PEG-20 Ester | 4,0000 |
| Lantte^{®} O | Cetearyl Alcohol | 1,0000 |
| PCL-Liquid | Cetearyl Ethylhexanoate, Isopropyl Myristate | 2,2500 |
| Xiameter^{®} PMX-0245 | Cyclopentasiloxane | 3,7500 |

| **Phase B** | | |
|---|---|---|
| Wasser | Water | 78,8500 |
| Natriumbenzoat | Sodium Benzoate | 0,1000 |

| **Phase C** | | |
|---|---|---|
| Glycerin 99,5 P. | Parfum | 5,0000 |
| Preservative Complex (ND Liquid) | Triethylene Glygol, Imidazolidinyl Urea, Methylparaben, | 0,9000 |

| **Phase D** | | |
|---|---|---|
| Natriumhydroxid 30% Lösung | Sodium Hydroxide | 0,1300 |

| **Phase E** | | |
|---|---|---|
| Beispiel-Mischung 2 | Parfum | 0,0100 |
| | Summe | 100,0000 |

Zur Herstellung der Phase B wird das Natriumbenzoat zu dem Wasser dazu gegeben und vermischt bis es vollständig gelöst ist. Dann wird die vorgemischte Phase C zu der Phase B gegeben und unter Rühren mittels eines Magnetrührers auf 75 °C erwärmt.

Die Bestandteile der Phase A werden separat vermischt und dann in einem Wasserbad erwärmt und homogenisiert. Unter Rühren (Rayneri = deflocculator oder mindestens eine Helix) wird die Phase A zu dem Gemisch aus Phase B und C gegeben. Danach wird dieses Gemisch unter Rühren (Helix) auf 30 °C abgekühlt.

Anschließend wird die Phase D hinzugefügt, um den pH-Wert auf 6,0 einzustellen. Danach wird die Phase E, das Parfüm hinzugefügt.

### Waschpulver

### Allzweckreiniger

**Tabelle 30: Allzweckreiniger**

| **Bestandteil** | **INCI** | **Gew.-%** |
|---|---|---|
| Entionisiertes Wasser | Water | 59,6000 |
| Mergal K9N | 5-Chloro-2-methyl-3-(2H)-isothiazolone und 2-methyl-3-(2H)-isothiazolone | 0,1000 |
| Trinatriurrcitrat Dihydrat | Trisodium citrate dihydrate | 3,0000 |
| Zetesol NL-2 | Fatty alcohol C12-14-sulfate, Sodium | 30,0000 |
| Imbentin C/125/055 | Fatty alcohol C12-15, 8EO | 5,0000 |
| Ethanol 96% | Ethanol | 2,0000 |
| Beispiel-Mischung 10 | Parfum | 0,3000 |
| | Summe | 100,0000 |

### Shampoo

**Tabelle 31: Shampoo**

| **Bestandteil** | **INCI** | **Gew.-%** |
|---|---|---|
| Entionisiertes Wasser | Water | 71,5000 |
| Plantacare PS 10 | Sodium Laureth Sulfate, Lauryl Glucoside | 20,0000 |
| Euperlan PK 771 | Glycol Distearate, Sodium Lauryl Sulfate, | 6,0000 |
| Dragocid Liquid | Phenoxyethanol, Methylparaben, Ethylparaben, | 0,5000 |
| Natriumchlorid | Sodium Chloride | 1,4000 |
| Citronensäure Monohydrat kristallin | Citric Acid | 0,1000 |
| Beispiel-Mischung 12 | Parfum | 0,5000 |
| | Summe | 100,0000 |

### Duschgel

### Transparente Deo-Stick (Formulierung A) bzw. Creme-Deo-Creme-Stick Formulierung B)

**Tabelle 33: Transparente Deo-Sticks**

| **Bestandteil** | **A Gew.-%** | **B Gew.-%** |
|---|---|---|
| Entionisiertes Wasser | 36,5500 | 54,8500 |
| Aluminium Zirconium Tetrachlorohydrat - Glycin Komplex | 25,0000 | 25,0000 |
| Dimethicon (10 Cst) | - | 5,0000 |
| Cyclopentasiloxan | - | 1,0000 |
| Petrolatum | 5,0000 | 5,0000 |
| Ozokerit | 1,0000 | - |
| Stearylalkohol | 12,0000 | - |
| 2-Butyloctansäure | 0,5000 | 0,5000 |
| Wachs | - | 1,2500 |
| PPG-14 Butylether | 9,0000 | - |
| Gehärtetes Rapsöl | - | 5,0000 |
| Siliciumdioxid | - | 1,0000 |
| Farnesol | 0,2500 | 0,2500 |
| Paraffinöl | 0,5000 | - |
| Hydriertes Rizinusöl (castor w ax) | 3,5000 | - |
| Talk | 4,0000 | - |
| Behenylalkohol | 0,2000 | - |
| d-Panthenyltriacetat | 1,0000 | - |
| Beispiel-Mischung 13 | 1,5000 | 1,1500 |
| Summe | 100,0000 | 100,0000 |

### Roll-on Antiperspirant

**Tabelle 34: Roll-on Antiperspirant**

| **Bestandteil** | **Gew.-%** |
|---|---|
| Entionisiertes Wasser | 72,9000 |
| Caprylyl Trimethicon (SilCare TM Silicone 31 M 50) | 0,3000 |
| Steareth-20 (GENAPOL TM HS 200) | 3,0000 |
| Steareth-2 (GENAPOL TM HS 020) | 1,5000 |
| Dicaprylyl Ether (Cetiol TM OE) | 2,0000 |
| Coco Caprylat/Caprat (Cetiol TM LC) | 2,0000 |
| Glycerin | 2,0000 |
| Glyceryl Stearat (Cutina TM GMS) | 2,0000 |
| Octyldodecanol (Eutanol TM G) | 1,0000 |
| Stearyl alkohol | 2,5000 |
| Aluminiumchlorohydrat gemäß Beispiel 1 der EP 1321431 | 10,0000 |
| Avocado Extract Persea Gratissima | 0,3000 |
| Beispiel-Mischung 12 | 0,5000 |
| Summe | 100,0000 |

### Aerosolspray

### Haarconditioner mit UV-Schutz

### Sonnenschutzspray

**Tabelle 37: Sonnenschutzspray**

| **Bestandteil** | **INCI** | **Gew.-%** |
|---|---|---|
| **Phase A** | | |
| Wasser, demineralisiert | Water (Aqua) | 68,9000 |
| Glycerin | Glycerin | 4,0000 |
| 1,3 Butylenglykol | Butylene Glycol | 5,0000 |
| D-Panthenol | Panthenol | 0,5000 |
| Lara Care A-200 | Galactoarabinan | 0,2500 |

| **Phase B** | | |
|---|---|---|
| Baysiloneöl M 10 | Dimethicone | 1,0000 |
| Edeta BD | Disodium EDTA | 0,1000 |
| Copherol 1250 | Tocopheryl Acetate | 0,5000 |
| Cetiol OE | Dicaprylyl Ether | 3,0000 |
| Neo Heliopan^{®} HMS | Homosalate | 5,0000 |
| Neo Heliopan^{®} AV | Ethylhexyl Methoxycinnamate | 6,0000 |
| Neo Heliopan^{®} 357 | Butyl Methoxydibenzoylmethane | 1,0000 |
| Corapan TQ | Diethylhexylnaphthalate | 2,0000 |
| Alpha Bisabolol | Bisabolol | 0,1000 |
| Pemulen TR-2 | Acrylates/C10-30 Alkyl Acrylate Crosspolymer | 0,2500 |

| **Phase C** | | |
|---|---|---|
| Phenoxyethanol | Phenoxyethanol | 0,7000 |
| Solbrol M | Methylparaben | 0,2000 |
| Solbrol P | Propylparaben | 0,1000 |

| **Phase D** | | |
|---|---|---|
| NaOH, 10 %ig | Sodium Hydroxide | 0,6000 |

| **Phase E** | | 0,5000 |
|---|---|---|
| Beispiel-Mischung 13 | Parfum | 0,2000 |
| Beispiel-Mischung 5 | Parfum | 0,1000 |
| | Summe | 100,0000 |

Zunächst wurde das Lara Care A-200 unter Rühren in den anderen Bestandteilen von Phase A gelöst.

Dann wurden alle Rohstoffe der Phase B ohne Pemulen eingewogen und die kristallinen Substanzen unter Erwärmen gelöst. Nach dem Lösen wurde das Pemulen eindispergiert. Danach wurde die Mischung der Phase B zu zur Mischung der Phase A gegeben und 1 Minute homogenisiert. Zum Schluss wurden die vorgemischten Phasen C-E hinzugefügt und nochmals für 1-2 Minuten mit dem Ultra Turrax homogenisiert.

### Sonnencreme mit Lichtschutzfaktor 40

**Tabelle 38: Sonnencreme mit Lichtschutzfaktor 40**

| **Bestandteil** | **INCI** | **Gew.-%** |
|---|---|---|
| **Phase A** | | |
| Dehymuls PGPH | Ethylhexyl Methoxycinnamate, Butyl | 5,0000 |
| Copherol 1250 | Ethylhexyl Isononanoate | 0,5000 |
| Permulgin 3220 | Tribehenin PEG-20 Ester | 0,5000 |
| Zinkstearat | Cetearyl Alcohol | 0,5000 |
| Tegosoft TN | Cetearyl Ethylhexanoate, Isopropyl Myristate | 10,0000 |
| Neo Heliopan^{®} E1000 | | 2,0000 |
| Neo Heliopan^{®} 303 | | 5,0000 |
| Neo Heliopan^{®} MBC | | 3,0000 |
| Zinkoxid neutral | Cyclopentasiloxane | 5,0000 |

| **Phase B** | | |
|---|---|---|
| Wasser | Water | 62,6000 |
| Glycerin | Glycerin | 0,1000 |
| Phenoxyethanol | Phenoxyethanol | 4,0000 |
| Solbrol M | Methylparaben | 0,7000 |
| Solbrol P | Propylparaben | 0,2000 |
| Magnesiumsulfat | Magnesium Sulfate | 0,1000 |

| **Phase C** | | 0,5000 |
|---|---|---|
| Beispiel-Mischung 13 | Parfum | 0,2000 |
| Beispiel-Mischung 4 | Parfum | 0,1000 |
| | Summe | 100,0000 |

Alle Bestandteile von Phase A wurden vermischt und auf ca. 85 °C erhitzt. Dann wurden die Bestandteile von

Phase B mit einander vermischt, mit Ausnahme des Zinkoxids, welches mit dem Ultra Turrax eindispergieren wird. Die Mischung der Bestandteile der Phase B wurde auf ca. 85 °C erhitzt. Danach wurde die Mischung der Phase B zur der Mischung der Phase A hinzugegeben und unter Rühren abgekühlt. Anschließend wurde Phase C hinzugefügt und homogenisiert.

### Sonnenschutzmilch

**Tabelle 39: Sonnenschutzmilch**

| **Bestandteil** | **INCI** | **Gew.-%** |
|---|---|---|
| **Phase A** | | |
| Dehymuls PGPH | Polyglyceryl-2 Dipolyhydroxystearate | 3,0000 |
| Bienenwachs 8100 | Beeswax | 1,0000 |
| Monomuls 90-0-18 | Glyceryl Oleate | 1,0000 |
| Zinkstearat | Zinc stearate | 1,0000 |
| Cetiol SN | Cetearyl Isononanoate | 5,0000 |
| Cetiol OE | Dicaprylyl Ether | 5,0000 |
| Tegosoft TN | C12-15 Alkyl Benzoate | 4,0000 |
| Vitamin E | Tocopherol | 0,5000 |
| Solbrol P | Propylparaben | 0,1000 |
| Neo Heliopan^{®} OS | Ethylhexyl Salicylate | 5,0000 |
| Neo Heliopan^{®} AV | Ethylhexyl Methoxycinnamate | 7,5000 |
| Uvinulä T150 | Ethylhexyl Triazone | 1,5000 |

| **Phase B** | | |
|---|---|---|
| Wasser | Water | 43,5500 |
| Trilon BD | Disodium EDTA | 0,1000 |
| Glycerin | Glycerin | 5,0000 |
| Solbrol M | Methylparaben | 0,2000 |
| Phenoxyethanol | Phenoxyethanol | 0,7000 |
| Neo Heliopan^{®} AP 10%ige Lösung, | Disodium Phenyl Dibenzirridazole Tetrasulfonate | 15,0000 |

| **Phase C** | | 0,5000 |
|---|---|---|
| Alpha Bisabolol | Bisabolol | 0,1000 |
| Beispiel-Mischung 12 | Parfum | 0,2500 |
| | Summe | 100,0000 |

### Transparente Gel-Zahnpasta

**Tabelle 40: Transparente Gel-Zahnpasta**

| **Bestandteil** | **Gew.-%** |
|---|---|
| Sorbitol 70% | 63,1750 |
| Entionisiertes Wasser | 11,3100 |
| Natriumcarboxymethylcellulose | 0,6000 |
| Natriumlaurylsulfat | 1,5000 |
| Natriummonofluorphosphat | 1,1400 |
| PEG 1500 (PEG 32) | 5,0000 |
| Pellitorin-Lösung (enthaltend 10% Pellitorin) | 0,0250 |
| Saccharin | 0,2000 |
| Sident 22 S (Verdickende Silica) | 8,0000 |
| Sident 9 (Abrasive Silica) | 8,0000 |
| Solbrol | 0,1500 |
| Trinatriurrphosphat | 0,1000 |
| Beispiel-Mischung 7 | 0,8000 |
| Summe | 100,0000 |

### Zahnpasta auf Phosphatbasis

**Tabelle 41: Zahnpasta auf Phosphatbasis**

| **Bestandteil** | **Gew.-%** |
|---|---|
| Entionisiertes Wasser | 36,3900 |
| Aerosil^{®} 200 (Silica) | 3,0000 |
| Dicalciurrphosphat-Dihydrat | 36,0000 |
| Glycerin | 20,0000 |
| Natriumcarboxymethylcellulose | 1,2000 |
| Natriumlaurylsulfat (Texapon) | 1,3000 |
| Natriurrmonofluorphosphat | 0,7600 |
| Saccharin | 0,2000 |
| Solbrol M (Natriumsalz) | 0,1500 |
| Beispiel-Mischung 7 | 1,0000 |
| Summe | 100,0000 |

### Zahnpasta mit Zink

**Tabelle 42: Zahnpasta mit Zink**

| **Bestandteil** | **Gew.-%** |
|---|---|
| Entionisiertes Wasser | 21,2000 |
| 1,2-Propylenglycol | 5,0000 |
| Abrasiv-Silica | 20,0000 |
| Farbstoff (1%ig in Wasser) | 0,5000 |
| Glycerin | 5,0000 |
| Na-gluconat | 1,5000 |
| Na-Saccharin | 0,5000 |
| Natriurrfluorid NaF | 0,5000 |
| Natriumhydroxid | 0,1000 |
| Natriurriaurylsulfat | 2,0000 |
| Polyethylenglycol | 1,0000 |
| Sorbitol, 70 %ig in Wasser | 36,0000 |
| Tetranatriumpyrophosphat | 2,5000 |
| Zinkcarbonat ZnCO3 | 1,0000 |
| Zinklactat | 2,0000 |
| Beispiel-Mischung 7 | 1,2000 |
| Summe | 100,0000 |

### Mundwasser

**Tabelle 43 : Mundwasser**

| **Bestandteil** | **Gew.-%** |
|---|---|
| Entionisiertes Wasser | 79,5800 |
| 1,2-Propylenglycol | 2,0000 |
| Benzoesäure | 0,1200 |
| Cremophor CO 40 | 1,0000 |
| Ethylalkohol 96% | 5,0000 |
| Glycerin | 8,0000 |
| L-Blue 5000 (1% in Wasser) | 0,1000 |
| Natriumsaccharin 450 | 0,0500 |
| Sorbitol 70% | 1,0000 |
| Wasserstoffperoxid (35% H2O2 in Wasser) | 3,0000 |
| Beispiel-Mischung 6 | 0,1500 |
| Summe | 100,0000 |

### Mundwasserkonzentrat

**Tabelle 44: Mundwasserkonzentrat**

| **Bestandteil** | **Gew.-%** |
|---|---|
| Entionisiertes Wasser | 49,6700 |
| Allantoin | 0,2000 |
| Colour L-Blue 5000 (1% in Wasser) | 0,0300 |
| Cremophor CO 40 | 5,0000 |
| Ethylalkohol 96% | 42,0000 |
| Natriumsaccharin 450 | 0,1000 |
| Beispiel-Mischung 6 | 3,0000 |
| Summe | 100,0000 |

### Kaugummi (mit Zucker und Zuckerfrei)

**Tabelle 45: Kaugummis (mit Zucker und Zuckerfrei)**

| **Bestandteil** | **A Gew.-%** | **B Gew.-%** |
|---|---|---|
| Puderzucker | 61,7500 | - |
| Sorbitol (in Pulverform) | - | 38,6000 |
| Acesulfam K | - | 0,1000 |
| Aspartam | - | 0,1000 |
| ErrulgumTM (Emulgator) | - | 0,3000 |
| Glucosesirup | 16,5000 | - |
| Glycerin | 0,5000 | 1,0000 |
| Gum Base (Kaugummibase) | 21,0000 | 30,0000 |
| Mannitol | - | 3,0000 |
| Palatinit | - | 9,5000 |
| Sorbitol 70%, in Wasser | - | 14,0000 |
| Xylitol | - | 2,0000 |
| Beispiel-Mischung 7 | 0,2500 | 1,4000 |
| Summe | 100,0000 | 100,0000 |

### Bonbon

**Tabelle 46: Bonbon**

| **Bestandteil** | **Gew.-%** |
|---|---|
| Wasser | 2,7500 |
| Zucker | 60,1000 |
| Glukosesirup | 36,9000 |
| Beispiel-Mischung 8 | 0,2500 |
| Summe | 100,0000 |

### Fine Fragrance

### Fortsetzung Tabelle Fine Fragrance 2

| **Stoff** | **Gew-%** |
|---|---|
| KAMPFER DL | 0,4064 |
| LAVANDINOEL GROSSO NAT. CENSO | 0,0830 |
| LIGUSTRAL | 0,0010 |
| LIMONEN D REIN | 0,0785 |
| LINALOOL | 0,2166 |
| LINALOOL BM | 2,5260 |
| LINALOOLOXID | 0,0008 |
| LINALYLACETAT | 0,1206 |
| MACISOEL ND | 0,0879 |
| MAJANTOL^{®} | 0,3624 |
| MAJORANOEL AEGYPT. | 0,0004 |
| MALTOL | 0,0004 |
| MANDARINE ALDEHYD 10% IN TEC 10% BB | 0,0879 |
| MANZANATE 10% DPG | 0,2636 |
| MENTHANYLACETAT | 1,1532 |
| MINTONAT | 0,4393 |
| MYRCEN | 0,0015 |
| NEROL 900 | 0,0016 |
| NEROLIDOL | 0,0033 |
| OCIMEN | 0,0022 |
| OCTANDIOL | 0,2197 |
| OCTANON-3 | 0,0012 |
| OCTENOL-1,3 | 0,0014 |
| ORANGENOEL 5X | 0,0001 |
| ORIGANUMOEL | 0,0549 |
| OXANTHIA 50% IN TEC | 0,0011 |
| PATCHOULIOEL ENTF. | 1,6254 |
| PELARGONSAEURE | 0,0002 |
| | |

### Fortsetzung Tabelle Fine Fragrance 2

| **Stoff** | **Gew-%** |
|---|---|
| PENTANDIOL | 0,2197 |
| PENTYLFORMIAT | 0,0003 |
| PFEFFEROEL SCHWARZ 10% BB | 0,2855 |
| EUCALYPTUSOEL | 0,0038 |
| PHENYLETHYLALKOHOL | 0,0033 |
| PHENYLETHYLALKOHOL | 0,1208 |
| PIMENTOEL REF. A | 0,0002 |
| PINEN ALPHA LAEVO NAT. | 0,2855 |
| PINEN BETA NAT. | 0,0878 |
| PINOACETALDEHYD | 0,0329 |
| PRECYCLEMONE B | 0,1318 |
| PROPANDIOL | 0,8786 |
| RHUBOFIX | 0,0011 |
| ROSENOXID | 0,0016 |
| ROSENOXID HIGH CIS | 0,0001 |
| ROSMARINOEL BM | 0,2636 |
| SANDRANOL^{®} | 0,6590 |
| STERNANISOEL ROH 10% TEC | 0,0002 |
| TERPINEN GAMMA | 0,0002 |
| TERPINENOL-4 NAT. | 0,0020 |
| TERPINEOL ALPHA | 0,0030 |
| TERPINEOL REIN | 0,1195 |
| TERPINOLEN DEXTRO | 0,0003 |
| TETRAHYDROGERANIOL | 0,0000 |
| TONALID | 2,7457 |
| TRIETHYLCITRAT | 0,0584 |
| VELOUTONE | 0,0011 |
| VERTOCITRAL | 0,1647 |
| Summe | 100,0000 |

### Bonbon, Zuckerfrei

**Tabelle 49: Bonbon**

| **Bestandteil** | **Gew.-%** |
|---|---|
| Isomalt | 95,0225 |
| Wasser | 2,2400 |
| Xylitol | 2,4000 |
| Sucralose | 0,0300 |
| Acesulfam K | 0,0500 |
| Citronensäure | 0,0500 |
| Pellitorin-Lösung (enthaltend 10% Pellitorin) | 0,0075 |
| Beispiel-Mischung 8 | 0,2000 |
| Summe | 100,0000 |

## Patentansprüche

1. Verwendung einer Stoffmischung enthaltend
(a) ein, zwei oder mehrere Monoterperne ausgewählt aus der Gruppe, die gebildet wird von Linalool, Geraniol, Freesiol und Nerolidol,
(b) mindestens zwei Lactone ausgewählt aus der Gruppe, die gebildet wird von gamma-Dodecalacton, gamma-Decalacton, delta-Decalacton und delta-Dodecalacton;
(c1) Ethanol; und
(c2) ein, zwei oder mehrere Lösungsmittel ausgewählt aus der Gruppe, die gebildet wird von Dipropylenglykol, Diethylphtalat, Propylenglykol, Isopropylmyristat, Isopropylpalmitat, Triethylcitrat, Triacetin, 1,2-Propandiol, 1,3-Propandiol, Propantriol, Pentandiol, Hexandiol, Octandiol, Decandiol, Dodecanol, 4-Hydroxyacetophenon, und Benzylbenzoat
zur Reduzierung und/oder Maskierung von unerwünschten Fehlnoten in oleochemischen Zubereitungen, wobei die unerwünschten Fehlnoten in der oleochemischen Zubereitungen durch
(i) Aldehyde der Formel (III) 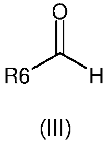 wobei R6 für einen gesättigten oder ungesättigten, linearen Kohlenwasserstoffrest mit 3 bis 10 Kohlenstoffatomen steht, und/oder
(ii) freie Fettsäuren der Formel (IV) 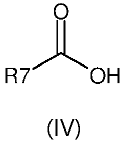 wobei R7 für einen linearen oder verzweigten, gesättigten Kohlenwasserstoffrest mit 3 bis 12 Kohlenstoffatomen steht, hervorgerufen werden.

2. Verwendung nach Anspruch 1, **dadurch gekennzeichnet, dass** die Stoffmischung weitere Aroma- bzw. Duftstoffe (Komponente d) enthalten kann, die ausgewählt sind aus der Gruppe bestehend aus 3-Phenylbutanal, Acetylmethylcarbinol, A-nethol, Anisylacetat, Dihydroeugenol, Linalylformiat, 2-Methyldecanal, 2-Benzyl-2-methylbut-3-ene nitrile, 3-Hexenylacetat, Styralylacetat, Belanis, Citronellal, Cinnamylacetat, Rhubafuran, beta-Ionon, Anther, Prenylacetat, 2-Phenylpropanal, 4-(4-Hydroxyphenyl)butan-2-on, Ethylphenoxyacetat, Isoralderin, gamma-Terpinen, Limonen, Neocyclocitral, Methyllavenderketon, Styralylpropionat, Phenylethylpropionat, Limonenal, 4-Isopentylcyclohexanol, 4-methyl-2-phenyl-3,6-dihydro-2H-py-ran/4-methylen-2-phenyl-tetrahydropyran, Hydrocitronitril, Phenoxanol, Isoamylphenylacetat, Damascon, Silvial, Nectaryl, Ambroxid, Acetylpyrazin, Trimethylpyrazin, Isoamylacetat, Cresylmethylether para, Filbertone, Cyclohexylacetat, Heliotropin, Acetophenon, Anisaldehyd, Methylacetophenon para, Veratraldehyd, Methylanisat und Vertoprenal,

3. Verwendung nach Anspruch 2, **dadurch gekennzeichnet, dass** die Komponente (d) ausgewählt ist aus der Gruppe, bestehend aus 3-Phenylbutanal, Acetylmethylcarbinol, 2-Methyldecanal, 2-Benzyl-2-methylbut-3-ene nitrile, 3-Hexenylacetat, Styralylacetat, Rhubafuran, Anther, Prenylacetat, Styralylpropionat, Isoamylphenylacetat, beta-Damascon, Isoamylacetat, Citronellol und Cyclohexylacetat.

4. Verwendung nach mindestens einem der Ansprüche 1 bis 3, **dadurch gekennzeichnet, dass** die Stoffmischung
(i) 0,0000001 bis 80 Gew.-% Komponente (a),
(ii) 0,0000001 bis 80 Gew.-% Komponente (b),
(iii) 0,0000001 bis 98 Gew.-% Komponente (c),
(iv) 0 bis 80 Gew.-% Komponente (d),
mit der Maßgabe enthält, dass sich die Mengenangaben mit gegebenenfalls weiteren Inhaltstoffen zu 100 Gew.-% addieren, bezogen auf das Gesamtgewicht der Stoffmischung.

5. Verwendung nach mindestens einem der Ansprüche 1 bis 4, **dadurch gekennzeichnet, dass** die Menge an Ethanol der Komponente (c1) kleiner als 30 Gew.-% ist, bezogen auf das Gesamtgewicht der Stoffmischung.

## Claims

1. Use of a substance mixture containing
(a) one, two or more monoterpenes selected from the group formed by linalool, geraniol, freesiol and nerolidol,
(b) at least two lactones selected from the group formed by gamma-dodecalactone, gamma-decalactone, delta-decalactone and delta -dodeca lactone;
(c1) ethanol; and
(c2) one, two or more solvents selected from the group formed by dipropylene glycol, diethyl phthalate, propylene glycol, isopropyl myristate, isopropyl palmitate, triethyl citrate, triacetin, 1,2-propanediol, 1,3-propanediol, propanetriol, pentanediol, hexanediol, octanediol, decanediol, dodecanol, 4-hydroxyacetophenone, and benzyl benzoate
for the reduction and/or masking of undesired off-notes in oleochemical preparations, wherein the undesired off-notes in the oleochemical preparations are caused by
(i) aldehydes of formula (III) 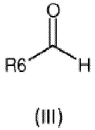 where R6 is a saturated or unsaturated, linear hydrocarbon radical having 3 to 10 carbon atoms, and/or
(ii) free fatty acids of formula (IV) 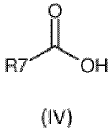 where R7 is a linear or branched, saturated hydrocarbon radical having 3 to 12 carbon atoms.

2. Use according to Claim 1, **characterized in that** the substance mixture can contain further aroma substances or fragrances (component d) which are selected from the group consisting of 3-phenylbutanal, acetylmethylcarbinol, anethole, anisyl acetate, dihydroeugenol, linalyl formate, 2-methyldecanal, 2-benzyl-2-methylbut-3-enenitrile, 3-hexenyl acetate, styralyl acetate, belanis, citronellal, cinnamyl acetate, rhubafuran, beta-ionone, anther, prenyl acetate, 2-phenylpropanal, 4-(4-hydroxyphenyl)butan-2-one, ethyl phenoxyacetate, isoraldeine, gamma-terpinene, limonene, neocyclocitral, methyl lavender ketone, styralyl propionate, phenethyl propionate, limonenal, 4-isopentylcyclohexanol, 4-methyl-2-phenyl-3,6-dihydro-2H-pyran/4-methylene-2-phe-nyltetrahydropyran, hydrocitronitrile, phenoxanol, isoamyl phenylacetate, damascone, silvial, nectaryl, ambroxide, acetyl pyrazine, trimethyl pyrazine, isoamyl acetate, para-cresyl methyl ether, filbertone, cyclohexyl acetate, heliotropin, acetophenone, anisaldehyde, para-methyl acetophenone, veratraldehyde, methyl anisate and vertoprenal.

3. Use according to Claim 2, **characterized in that** component (d) is selected from the group consisting of 3-phenylbutanal, acetylmethylcarbinol, 2-methyldecanal, 2-benzyl-2-methylbut-3-enenitrile, 3-hexenyl acetate, styralyl acetate, rhubafuran, anther, prenyl acetate, styralyl propionate, isoamyl phenylacetate, beta-damascone, isoamyl acetate, citronellol and cyclohexyl acetate.

4. Use according to at least one of Claims 1 to 3, **characterized in that** the substance mixture contains
(i) 0.0000001% to 80% by weight of component (a),
(ii) 0.0000001% to 80% by weight of component (b),
(iii) 0.0000001% to 98% by weight of component (c),
(iv) 0% to 80% by weight of component (d),
with the proviso that the quantitative data with optionally further ingredients add up to 100% by weight, based on the total weight of the substance mixture.

5. Use according to at least one of Claims 1 to 4, **characterized in that** the amount of ethanol of component (c1) is less than 30% by weight, based on the total weight of the substance mixture.

## Revendications

1. Utilisation d'un mélange de substances contenant
(a) un, deux ou plusieurs monoterpènes choisis dans le groupe formé par le linalool, le géraniol, le freesiol et le nérolidol,
(b) au moins deux lactones choisies dans le groupe formé par la gamma-do-décalactone, la gamma-décalactone, la delta-décalactone et la delta-do-décalactone ;
(c1) de l'éthanol ; et
(c2) un, deux ou plusieurs solvants choisis dans le groupe qui est formé par le di-propylèneglycol, le phtalate de diéthyle, le propylèneglycol, le myristate d'iso-propyle, le palmitate d'isopropyle, le citrate de triéthyle, la triacétine, le 1,2-propanediol, le 1,3-propanediol, le propanetriol, le pentanediol, l'hexanediol, l'octanediol, le décanediol, le dodécanol, la 4-hydroxyacétophénone et le benzoate de benzyle,
pour réduire et/ou masquer des odeurs étrangères indésirables dans des préparations oléochimiques, les odeurs étrangères indésirables étant dues, dans les préparations oléochimiques, à
(i) des aldéhydes de formule (III) 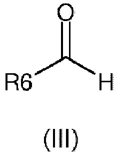 dans laquelle R6 représente un radical hydrocarboné linéaire saturé ou insaturé ayant 3 à 10 atomes de carbone, et/ou
(ii) des acides gras libres de formule (IV) 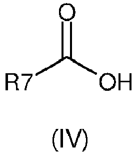 dans laquelle R7 représente un radical hydrocarboné saturé linéaire ou ramifié ayant 3 à 12 atomes de carbone.

2. Utilisation selon la revendication 1, **caractérisée en ce que** le mélange de substances peut contenir d'autres arômes ou parfums (composant d), qui sont choisis dans le groupe consistant en les composants suivants : 3-phénylbutanal, acétylméthylcarbinol, anéthol, acétate d'anisyle, dihydroeugénol, formiate de linalyle, 2-méthyldécanal, 2-benzyl-2-méthylbut-3-ène nitrile, acétate de 3-hexényle, acétate de styralyle, bélanis, citronellal, acétate de cinnamyle, rhubafurane, bêta-ionone, anthère, acétate de prényle, 2-phénylpropanal, 4-(4-hydroxyphényl)butan-2-one, phénoxya-cétate d'éthyle, isoraldérine, gamma-terpinène, limonène, néocyclocitral, méthyllav-endercétone, propionate de styralyle, propionate de phényléthyle, limonénal, 4-isopentylcyclohexanol, 4-méthyl-2-phényl-3,6-dihydro-2H-pyrane/4-méthylène-2-phényl-tétrahydropyrane, hydrocitronitrile, phénoxanol, phénylacétate d'isoamyle, damascone, silvial, nectaryl, ambroxyde, acétylpyrazine, triméthylpyrazine, acétate d'isoamyle, crésylméthyléther para, filbertone, acétate de cyclohexyle, héliotropine, acétophénone, anisaldéhyde, méthylacétophénone para, vératraldéhyde, anisate de méthyle et Vertoprenal.

3. Utilisation selon la revendication 2, **caractérisée en ce que** le composant (d) est choisi dans le groupe consistant en les composants 3-phénylbutanal, acétylméthylcarbinol, 2-méthyldécanal, 2-benzyl-2-méthylbut-3-ène nitrile, acétate de 3-hexényle, acétate de styralyle, rhubafurane, anthère, acétate de prényle, propionate de styralyle, phénylacétate d'isoamyle, bêta-damascone, acétate d'isoamyle, citronellol et acétate de cyclohexyle.

4. Utilisation selon au moins l'une des revendications 1 à 3, **caractérisée en ce que** le mélange de substances contient
(i) 0,0000001 à 80 % en poids du composant (a),
(ii) 0,0000001 à 80 % en poids du composant (b),
(iii) 0,0000001 à 98 % en poids du composant (c),
(iv) 0 à 80 % en poids du composant (d),
à la condition que les indications de quantité se complètent à 100 % en poids, avec éventuellement d'autres constituants, par rapport au poids total du mélange de substances.

5. Utilisation selon au moins l'une des revendications 1 à 4, **caractérisée en ce que** la quantité d'éthanol du composant (c1) est inférieure à 30 % en poids, par rapport au poids total du mélange de substances.
